(19) **Europäisches Patentamt**
**European Patent Office**
**Office européen des brevets**

(11) **EP 2 433 557 A1**

(12) **EUROPÄISCHE PATENTANMELDUNG**

(43) Veröffentlichungstag:
28.03.2012 Patentblatt 2012/13

(51) Int Cl.:
*A61B 5/024* (2006.01)  *G06F 19/00* (2011.01)

(21) Anmeldenummer: 11179130.7

(22) Anmeldetag: 29.08.2011

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 27.09.2010 US 386597 P

(71) Anmelder: BIOTRONIK SE & Co. KG
12359 Berlin (DE)

(72) Erfinder:
• Kirchner, Jens
91052 Erlangen (DE)
• Rockstroh, Christian
90443 Nürnberg (DE)
• Krämer, Thomas
90425 Nürnberg (DE)

(74) Vertreter: Lindner-Vogt, Karin L.
Biotronik SE & Co. KG
Corporate Intellectual Properties
Woermannkehre 1
12359 Berlin (DE)

(54) **Verfahren und Anordnung zur Ermittlung von phasenspezifischen Parametern einer physiologischen Größe sowie ein entsprechendes Computerprogramm und ein entsprechendes computerlesbares Speichermedium**

(57) Die Erfindung betrifft ein Verfahren und eine Anordnung zur Ermittlung von phasenspezifischen Parametern einer physiologischen Größe sowie ein entsprechendes Computerprogramm und ein entsprechendes computerlesbares Speichermedium, welche insbesondere einsetzbar sind, um Parameter von physiologischen Größen zu ermitteln, die der zirkadianen Variation unterworfen sind.

Hierfür wird vorgeschlagen, phasenspezifische Parameter einer physiologischen Größe X(t) dadurch zu ermitteln, dass zumindest für einen Teil der in einem vorgebbaren Zeitraum liegenden Werte x jeweils ein Mittelwert g(x|τ) aus solchen Werten X(t + τ) gebildet wird, für deren Vorgänger X(t) = x gilt, wobei τ einen zeitlichen Abstand beschreibt, und die phasenspezifischen Parametern durch Auswertung des Mittels g(x|τ) ermittelt werden.

Fig. 4

EP 2 433 557 A1

**Beschreibung**

Die Erfindung betrifft ein Verfahren und eine Anordnung zur Ermittlung von phasenspezifischen Parametern einer physiologischen Größe sowie ein entsprechendes Computerprogramm und ein entsprechendes computerlesbares Speichermedium, welche insbesondere einsetzbar sind, um Parameter von physiologischen Größen zu ermitteln, die der zirkadianen Variation unterworfen sind.

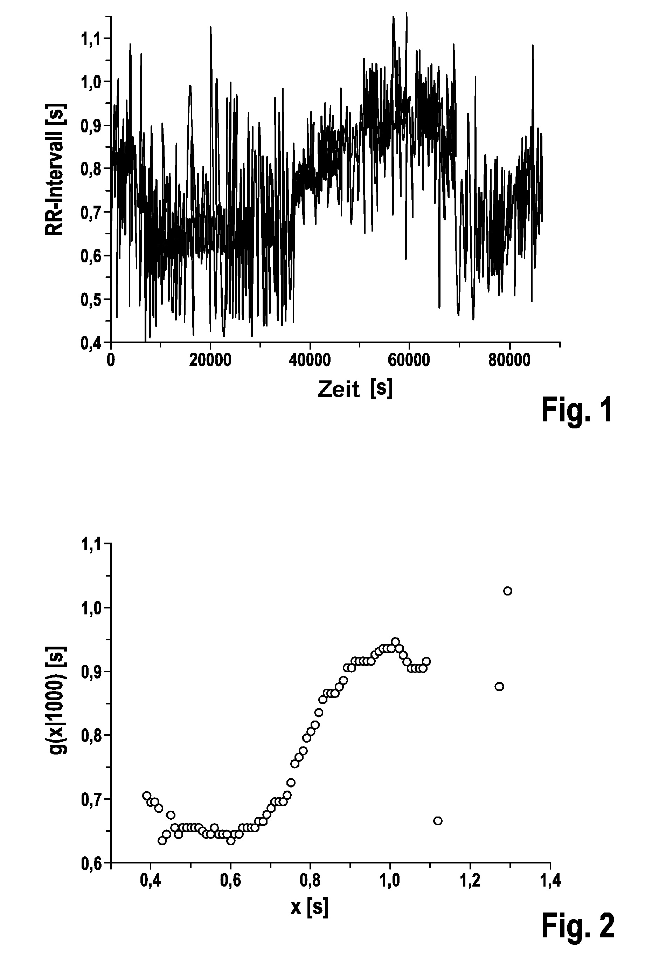

Die zirkadiane Variation, d. h. die durch den Tag-Nacht-Rhythmus eines Patienten bedingte periodische Schwankung einer physiologischen Größe, ist ein Phänomen, das sich bei ausreichend langen, insbesondere über 24h gemessenen Daten bei allen Menschen mehr oder weniger stark ausgeprägt findet (s. Figur 1). Es spiegelt sich darin die Tagesaktivität des Patienten im Vergleich zum nächtlichen Ruhezustand wider und damit auch indirekt die Leistungsfähigkeit des Herzkreislaufsystems. Dies legt nahe, dass Parameter, die die zirkadiane Variation quantitativ beschreiben, potentielle Indikatoren für die Ausbildung oder Verschlechterung kardialer Erkrankungen darstellen.

Gleichzeitig ist festzustellen, dass die Zeiten, in denen ein Patient schläft bzw. wach ist, deutlich unterschiedliche physiologische Situationen für das Herzkreislaufsystem darstellen. Bei der Datenanalyse, insbesondere bei der Berechnung diagnostisch relevanter Parameter, sollten diese Zeitabschnitte daher getrennt behandelt werden. Dazu ist eine vorgeschaltete Identifizierung der Tag-Nacht-Grenzen notwendig.

Im Folgenden soll eine Methode zur Bestimmung der Mittelwerte von Tag- und Nachtabschnitten einer der zirkadianen Variation unterworfenen physiologischen Größe, insbesondere der Herzrate, vorgestellt werden. Mit deren Hilfe lassen sich sowohl diagnostisch wertvolle Größen zur Erkennung und Überwachung von Herzkrankheiten berechnen als auch eine Trennung der Zeitintervalle durchführen, in denen der Patient wach ist bzw. schläft, um so für andere Methoden der Datenanalyse geeignete Datensegmente auszuwählen. Weiterhin können z. B. implantierbare medizinische Geräte (Herzschrittmacher, implantierbare Kardioverter/Defibrillatoren, implantierbare Geräte zur Patientenüberwachung und ähnliche), die mit diesem Algorithmus ausgestattet sind, ihre Funktionen patientenspezifisch auf die unterschiedlichen Anforderungen in der Tag- bzw. Nachtphase abstimmen und so die Lebensqualität des Patienten weiter verbessern.

Aus dem Stand der Technik ist bekannt, die jeweiligen Mittelwerte zu festen Zeiten bei kurzen Messdauern zu bestimmen. Tag- und Nachtphasen werden dabei aufgrund fester zeitlicher Grenzen definiert oder es werden Patientenprotokolle genutzt.

Die Bestimmung der Mittelwerte zu fixierten Zeiten weisen drei Nachteile auf:

1. Sie werden beeinträchtigt durch individuelle/zeitweilige Abweichungen vom eingestellten Rhythmus. Z. B. wird die Messungen der Nacht-Herzrate um 3 Uhr dadurch beeinflusst, dass der jeweilige Patient erst vor kurzem eingeschlafen ist, gerade eine Tiefschlafphase hat, bald aufstehen wird oder gerade auf die Toilette geht.
2. Sie verwenden nur eine kurze Messdauer. Dadurch werden sie anfälliger gegenüber Störungen wie im oben genannten Beispiel.
3. Sie stellen per definitionem keine Quantifizierung der zirkadianen Variation im Sinne einer Bestimmung von Schlaf- und Wachzeiten zur Verfügung

In einem extremen Beispiel wird die Herzrate je um 3 Uhr nachts und nachmittags gemessen, zu Zeiten, in denen der spezifische Patient für kurze Zeit aufgewacht ist bzw. ein Nachmittagsnickerchen hält. Detektiert würde dann eine völlige Inaktivität des Patienten, da sich Tag- und Nacht-Herzrate kaum unterscheiden. Entsprechendes gilt für die Datenverarbeitung, die z. B. Daten in einem festgelegten Zeitabschnitt als Nachtphase analysiert. Dieses Vorgehen verwirft einen Teil der eigentlich vorhandenen Daten oder/und läuft Gefahr, auch Daten aus der Wachphase mit zu integrieren.

Die Verwendung von Patientenprotokollen ist aus folgenden Gründen nachteilig:

1. Sie hängen von der Sorgfalt und mentalen Verfassung des Patienten ab und sind daher unsicher.
2. Sie stellen eine Unannehmlichkeit für den Patienten dar.

Es ist daher Aufgabe der Erfindung, ein Verfahren und eine Anordnung zur Ermittlung von phasenspezifischen Parametern einer physiologischen Größe sowie ein entsprechendes Computerprogramm und ein entsprechendes computerlesbares Speichermedium bereitzustellen, welche die Nachteile der herkömmlichen Lösungen vermeiden und insbesondere eine Quantifizierung der zirkadianen Variation ermöglichen ohne Vorannahmen beispielsweise über Anzahl oder Lage der Phasen der zirkadianen Variation.

Diese Aufgabe wird erfindungsgemäß durch die Merkmale in den Ansprüchen 1, 11 und 13 bis 15 gelöst. Zweckmäßige Ausgestaltungen der Erfindung sind in den Unteransprüchen enthalten.

Ein besonderer Vorteil des erfindungsgemäßen Verfahrens besteht darin, dass Parameter einer physiologischen Größe, wie z. B. der Herzrate, der Atmung, (bio)chemischer Signale (z. B. Konzentrationsschwankungen von Biomarkern) oder dergleichen, für verschiedene Phasen der physiologischen Größe X(t) bestimmt werden können, ohne vorherige Kenntnis oder bei nur unzureichender Kenntnis der Anzahl und/oder Grenzen der Phasen der Größe X(t). Dies wird dadurch erreicht, dass eine Funktion $g(x|\tau)$ von mittleren Zielzuständen in Abhängigkeit eines um $\tau$ früheren Ausgangszustands definiert wird: $g(x|\tau)= \langle X(t+\tau)\rangle_{X(t)=x}$. Dabei wird das zeitliche Mittel aller derjenigen Werte $X(t+\tau)$ gebildet, deren Vorgänger X(t) den Wert x hatte. Da es sich bei der physiologischen Größe X(t) beispielsweise auch um die Herzrate handeln kann, kann die Variable t im Folgenden neben der Zeit, gemessen in Sekunden, auch die Schlagnummer bezeichnen. Gleiches gilt für die Verzögerungszeit $\tau$ in Sekunden, die auch als Abstand in Schlägen aufgefasst werden kann. Allgemein beschreibt t einen Zeitpunkt oder ein Ereignis der physiologischen Größe X(t) wie beispielsweise einen Herzschlag bzw. die Nummer eines Ereignisses und $\tau$ eine Zeitspanne bzw. eine Anzahl von Ereignissen. Durch Auswertung der Funktion $g(x|\tau)$ lassen sich Parameter gewinnen, die im Zusammenhang mit verschiedenen Phasen der Größe X(t) stehen, insbesondere im Zusammenhang mit der zirkadianen Variation oder mit anderen physiologisch ausgezeichneten Zeitabschnitten. Eine bevorzugte Ausführungsform sieht vor, weitere sekundäre Parameter aus dem Sensorsignal X(t) selbst oder in Kombination mit weiteren Messgrößen zu bestimmen.

Die so gewonnenen Parameter können beispielsweise als Indikatoren für kardiale Erkrankungen, z. B. Herzinsuffizienz, oder als Indikatoren für Schlafstörungen genutzt werden. Die so gewonnenen Parameter können darüber hinaus auch der Identifikation von Datensegmenten entweder des genannten Sensorsignals X(t) oder einer anderen Messgröße zur weiteren Datenverarbeitung dienen.

Eine bevorzugte Ausführungsform der Erfindung sieht vor, dass die Ermittlung von phasenspezifischen Parametern die Ermittlung mindestens eines phasenspezifischen Mittelwertes $\xi$, den die Größe X(t) in einer bestimmten Phase annimmt, die Ermittlung von Grenzen und/oder der Länge der Phasen umfasst. Dadurch erhält man Parameter zur Quantifizierung der zirkadianen Variation als Mittel zur Erkennung und Überwachung kardialer Erkrankungen. Insbesondere kann durch die mit dem erfindungsgemäßen Verfahren gewonnenen Parameter auch eine Quantifizierung des Schlafverhaltens eines Patienten vorgenommen werden, anhand der Schlafstörungen erkannt und überwacht werden können.

In einer bevorzugten Ausführungsform der Erfindung ist vorgesehen, dass die Werte der Herzrate ausgewertet werden. Beispiele für daraus ermittelte Parameter sind:

- Tag-Mittelwert $\xi_1$ (der Tagphase)
- Nacht-Mittelwert $\xi_2$ (der Nachtphase)
- Differenz $(\xi_2 - \xi_1)$ zwischen Tag- und Nacht-Mittelwert
- Anzahl der Schläge in der Nachtphase, d. h. Anzahl der Schläge oberhalb eines Schwellwerts, z.B. $\#\{X(t) | X(t) > \xi_2 - \frac{1}{4}\cdot(\xi_2-\xi_1)\}$
- Anzahl der Schläge in der Tagphase
- Länge der Nachtphase
- Länge der Tagphase

Es ist weiter von Vorteil, wenn eine Klassifikation der Werte der Größe X(t) erfolgt, indem die Werte von X(t) zumindest einem Teil der Phasen zugeordnet werden. Diese Zuordnung kann auf Basis der Mittelwerte $\xi$ der einzelnen Phasen erfolgen, beispielsweise indem durch einen oder mehrere der Mittelwerte ein oder mehrere Schwellwerte definiert werden, und die Werte X(t) in Abhängigkeit ihrer Relation zu dem (den) Schwellwert(en) einer Phase zugeordnet werden.

Eine weitere bevorzugte Ausführungsform der Erfindung sieht vor, dass die Lage der Phasen, d. h. beispielsweise die Grenzen und/oder Längen der Phasen, unter Auswertung der klassifizierten Werte von X(t) ermittelt wird. Die Ermittlung der Lage der Phasen kann insbesondere eine Optimierung umfassen. In einer bevorzugten Ausführungsform ist hierfür vorgesehen, dass beispielsweise die Grenzen einer Phase dadurch bestimmt werden, dass die Anzahl der (beispielsweise durch eine Klassifikation) einer ersten Klasse zugeordneten Werte innerhalb der ersten Phase und die Anzahl der einer zweiten Klasse zugeordneten Werte vor und nach der ersten Phase durch Variation der Phasengrenzen maximiert werden. Eine andere bevorzugte Ausführungsform sieht vor, dass die Grenzen einer Phase dadurch bestimmt werden, dass für einen Beobachtungszeitraum, wie beispielsweise 24 Stunden, Zeitintervalle einer vorgegebenen Länge daraufhin betrachtet werden, ob die Anzahl der von einem Zeitintervall umfassten Werte X(t), die einer ersten Phase zugeordnet wurden, in einem bestimmten vorgebbaren Verhältnis zu der von diesem Zeitintervall umfassten Anzahl von Werten X(t) stehen, die einer zweiten (oder dritten) Phase zugeordnet wurden, und anhand dieses Verhältnisses das Zeitintervall einer Phase zugeordnet wird. In dem beispielhaften Fall der Herzrate X(t) kann es sich bei der ersten und zweiten Phase um die Tag- bzw. Nachtphase handeln, und die Klassifizierung der Schläge in Tag- bzw. Nachtschläge vorgenommen werden, wie es weiter unten näher erläutert wird.

Ein besonderer Vorteil der Erfindung liegt darin, dass neben der zirkadianen Variation auch kleinere Strukturen von physiologischen Größen ermittelt werden können, wie z. B. Tiefschlafphasen, REM-Schlaf, Mittagsschlaf oder Zeiten hoher Belastung. Dies wird erreicht durch eine rekursive Auswertung der Funktion $g(x|\tau)$ der mittleren Zielzustände. Dabei werden in einem ersten Schritt beispielsweise Grenzen und Länge einer ersten Menge von Phasen der Größe X(t) durch das erfindungsgemäße Verfahren ermittelt, indem die Funktion $g(x|\tau)$ für einen ersten vorgegebenen Zeitraum, beispielsweise einen Tag, gebildet und ausgewertet wird. In einem zweiten Schritt wird das Verfahren auf eine oder parallel mehrere im ersten Schritt ermittelte Phasen angewandt und dabei für jede der nun untersuchten Phasen eine neue Funktion $g(x|\tau)$ gebildet. Vorzugsweise wird dabei die Dauer (die Anzahl) $\tau$ der Länge der untersuchten Phasen und/oder der zu untersuchenden Ereignisse, wie z. B. Tiefschlaf-, REM-Schlafphasen oder dergleichen, angepasst. Vorzugsweise wird $\tau$ so gewählt, dass $\tau$ kleiner als die Dauer der zu untersuchenden Ereignisse ist.

Eine weitere bevorzugte Ausführungsform der Erfindung sieht vor, dass die Funktion $g(x|\tau)$ durch eine stückweise lineare Funktion $y_1(x)$ approximiert wird. In einer bevorzugten Ausführungsform ist dabei vorgesehen, dass die Funktion $y_1(x)$ Bereiche umfasst, in denen die Funktion $y_1(x)$ einen konstanten Verlauf aufweist. Die Bereiche konstanten Verlaufs werden durch Bereiche verbunden, in denen die Funktion $y_1(x)$ einen linear ansteigenden oder absteigenden Verlauf aufweist. Als vorteilhaft erweist es sich, wenn die Funktion $y_1(x)$ wie folgt definiert wird:

$$y_1(x) = \begin{cases} \xi_1 & \text{falls } x < \xi_1 + dx, \\ x & \text{falls } \xi_1 + dx < x < \xi_2 - dx, \\ \xi_2 & \text{falls } \xi_2 - dx < x \end{cases}$$

mit freien Parametern $dx$, $\xi_1$ und $\xi_2$. Vorzugsweise wird $dx = 0$ gewählt; $dx > 0$ kann sich jedoch als vorteilhaft erweisen, um die Funktion $y(x)$ besser an den Verlauf der Funktion $g(x|\tau)$ anzupassen.

Eine weitere bevorzugte Ausführungsform der Erfindung sieht vor, dass die Funktion $g(x|\tau)$ durch eine sigmoidale Funktion $y_s(x)$ approximiert wird. Dem Fachmann erschließen sich für $g(x|\tau)$ weitere Fitfunktionen.

Um beispielsweise Artefakte zu vermeiden, die durch Werte der Funktion $g(x|\tau)$ verursacht werden, die auf einer geringen Datenbasis beruhen, erweist sich weiter als vorteilhaft den $\chi^2$-Wert zu gewichten. In einer bevorzugten Ausführungsform ist vorgesehen, den $\chi^2$-Wert in Abhängigkeit der Häufigkeitsverteilung $P(x) = \#\{X(t)=x\} / \#\{X(t)\}$ der Werte von $X(t)$ zu gewichten. Dabei und im Folgenden bezeichnet "#" die Anzahl der Elemente in den zugehörigen Mengen. Als vorteilhaft erweist es sich weiter, wenn durch eine Gewichtung die Dominanz großer Werte von $g(x|\tau)$ vermieden wird. Dies kann beispielsweise erreicht werden, indem oberhalb einer bestimmten Häufigkeit $P(x)$ die Gewichte kleiner als die Häufigkeit $P(x)$, vorzugsweise konstant, gewählt werden. Als vorteilhaft kann es sich auch erweisen, $\chi^2$ nur für einen eingeschränkten Definitionsbereich von $g(x|\tau)$ zu berechnen.

Eine Anordnung nach der Erfindung weist mindestens einen Chip und/oder Prozessor auf und ist derart eingerichtet, dass ein Verfahren zur Ermittlung von phasenspezifischen Parametern einer physiologischen Größe $X(t)$ ausführbar ist, wobei zumindest für einen Teil der in einem vorgebbaren Zeitraum liegenden Werte $x$ jeweils ein Mittelwert $g(x|\tau)$ aus solchen Werten $X(t + \tau)$ gebildet wird, für deren Vorgänger $X(t) = x$ gilt, wobei $\tau$ einen zeitlichen Abstand beschreibt, und die phasenspezifischen Parameter durch Auswertung des Mittels $g(x|\tau)$ ermittelt werden.

In einer bevorzugten Ausführungsform der Erfindung ist vorgesehen, dass die Anordnung einen Sensor zur Messung einer physiologischen Größe $X(t)$, z. B. der Herzrate, umfasst. Darüber hinaus umfasst die Anordnung vorzugsweise eine Einheit zur Datenauswertung, die als Teil eines Implantats im Körper eines Patienten oder als externes Gerät realisiert sein kann. Bei dem Implantat kann es sich beispielsweise um einen Herzschrittmacher, einen Defibrillator (ICD: Intra Cardiac Defibrillator) oder um ein Gerät zur Patientenüberwachung, z.B. einen Dekompensationsprädiktor (Worsening of Heart Failure Predictor WHFP), handeln. Ebenso kann das Gerät zur Patientenüberwachung entweder gänzlich als externes Gerät realisiert sein oder sich aus einer oder mehreren implantierbaren und einer oder mehreren externen Komponenten zusammensetzen, die in geeigneter Weise, z.B. elektrisch, oder mittels Magnetspule bzw. Telemetrie, ständig oder zeitweise miteinander verbunden sind.

Die Datenauswertung umfasst dabei vorzugsweise die Bestimmung der Funktion der mittleren Zielzustände

$$g(x|\tau) = \langle X(t+\tau) \rangle_{X(t)=x}$$

in Abhängigkeit des um $\tau$ früheren Ausgangszustandes $x$, d. h. das zeitliche Mittel aller derjenigen Werte $X(t+\tau)$, deren Vorgänger $X(t)$ (der um $\tau$ Schläge frühere Zyklus) den Wert $x$ hatte. Aus den so gewonnenen Daten können nun Parameter bestimmt werden, die im Zusammenhang mit der zirkadianen Variation oder anderen physiologisch ausgezeichneten Zeitabschnitten stehen. Optional können auch weitere sekundäre Parameter bestimmt werden, entweder aus dem Sensorsignal X(t) selbst oder in Kombination mit einer oder mehreren Messgrößen.

Die Datenauswertung zur Ermittlung der Funktion g(x|τ) kann dabei kontinuierlich erfolgen, d. h. mit fortschreitendem Erfassen des Sensorsignals X(t) aktualisiert werden, oder nach Abschluss der Erfassung des Sensorsignals X(t) durch Auswertung der gesamten erfassten Datenmenge ermittelt werden. Die kontinuierliche Aktualisierung hat den Vorteil, dass relativ geringe Rechenressourcen für die Aktualisierung benötigt werden, während bei einer Ermittlung von g(x|τ) nach Abschluss des Messvorgangs u.U. sehr große Datenmengen ausgewertet werden müssen.

Durch die Erfindung wird eine Reduktion der zu übertragenden Datenmenge ermöglicht, insbesondere dann, wenn die physiologischen Daten mit einem Implantat erfasst, mittels Telemetrie übertragen und in einem externen Gerät verarbeitet werden. Durch die Berechnung der Funktion g(x|τ) im Implantat reduziert sich die Anzahl der zu speichernden Zyklen von z. B. der Herzrate im Implantat auf τ Zyklen. Zusätzlich müssen pro Bin dessen g-Wert, der Wertebereich sowie die Anzahl der bisher zum g-Wert beitragenden Datenpunkte gespeichert werden. Bei einer Abdeckung des Messbereichs mit 1000 Bins und einer Verzögerungszeit von τ = 1000 ergibt sich ein Speicherbedarf von 4000 Datenpunkten im Implantat. Davon werden (bei fest eingestellten Bingrenzen) einzig die 1000 Datenpunkte des g-Plots an ein externes Gerät übertragen. Gegenüber einer 24h-Messung mit typischerweise ca. 100 000 Datenpunkten stellt dies eine wesentliche Reduktion der zu speichernden und zu übertragenden Datenmenge dar.

Die mit Hilfe der Erfindung gewonnenen Parameter und/oder Informationen, z. B. über das Schlafverhalten eines Patienten, werden in einer bevorzugten Ausführungsform der Erfindung genutzt, um Funktionen der Anordnung, insbesondere eines Herzschrittmachers, ICDs und/oder Herzmonitors (WHF-Prädiktors) vorzugsweise automatisch individuell anzupassen.

Ein Computerprogramm zur Ermittlung von phasenspezifischen Parametern einer physiologischen Größe X(t) ermöglicht es einer Datenverarbeitungseinrichtung, nachdem es in den Speicher der Datenverarbeitungseinrichtung geladen worden ist, phasenspezifische Parameter zu ermitteln, wobei zumindest für einen Teil der in einem vorgebbaren Zeitraum liegenden Werte x jeweils ein Mittelwert g(x|τ) aus solchen Werten X(t+τ) gebildet wird, für deren Vorgänger X(t) = x gilt, wobei τ einen zeitlichen Abstand beschreibt, und die phasenspezifischen Parameter durch Auswertung des Mittels g(x|τ) ermittelt werden.

In einer weiteren bevorzugten Ausführungsform der Erfindung ist vorgesehen, dass das erfindungsgemäße Computerprogramm modular aufgebaut ist, wobei einzelne Module auf verschiedenen Datenverarbeitungseinrichtungen installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren zur Ermittlung von phasenspezifischen Parametern einer physiologischen Größe X(t) durchzuführen, ist vorgesehen, ein computerlesbares Speichermedium einzusetzen, auf dem ein Programm gespeichert ist, das es einer Datenverarbeitungseinrichtung ermöglicht, nachdem es in den Speicher der Datenverarbeitungseinrichtung geladen worden ist, phasenspezifische Parameter zu ermitteln, wobei zumindest für einen Teil der in einem vorgebbaren Zeitraum liegenden Werte x jeweils ein Mittelwert g(x|τ) aus solchen Werten X(t + τ) gebildet wird, für deren Vorgänger X(t) = x gilt, wobei τ einen zeitlichen Abstand beschreibt, und die phasenspezifischen Parameter durch Auswertung des Mittels g(x|τ) ermittelt werden.

Die Erfindung stellt ein effizientes Verfahren zur Ermittlung von phasenspezifischen Parametern einer physiologischen Größe X(t) bereit, wobei die oben genannten Nachteile des Standes der Technik vermieden werden in dem Sinne, dass sie von individuellen bzw. vereinzelt auftretenden Schwankungen des typischen Tag-Nacht-Rhythmus nicht beeinträchtigt wird. Das liegt daran, dass sie keine Vorannahmen über Anzahl, Lage und (zumindest weitestgehend) die Länge der Wach- und der Schlafphasen macht. Ferner verzichtet sie auf die Verwendung von Patientenprotokollen.

In Tests hat sich außerdem gezeigt, dass die Erfindung sowohl bei schrittmacherabhängigen Patienten als auch bei Probanden ohne Implantat funktioniert. Das Vorliegen von Abschnitten ohne intrinsischen Rhythmus beeinträchtigt die Methode also nicht.

Ein weiterer Vorteil der Methode besteht darin, dass sie die bereits in Implantaten vorhandenen Sensoren verwenden kann und nicht auf die Integration eines zusätzlichen Messsystems angewiesen ist. Insbesondere ist der Einsatz von Beschleunigungs- oder Bewegungssensoren nicht notwendig.

Die Erfindung könnte in einen Dekompensationsprädiktor integriert werden, zum einen als Mittel zur Datenvorverarbeitung für schon verwendete oder andere noch zu implementierende Analysemethoden oder zum anderen mit den oben beschriebenen, aus der zirkadianen Variation abgeleiteten Parametern als Indikatoren für eine Verschlechterung/Entstehung von CHF.

Neben kardialen Erkrankungen lassen sich auch verschiedene Schlafstörungen detektieren und überwachen, insbesondere Schlafapnoe.

Durch die Erfindung wird ermöglicht, Mittelwerte einer physiologischen Größe zu bestimmen, insbesondere der Herzrate, aber auch z. B. der Atmung, (bio)chemischer Signale (z. B. Konzentrationsschwankungen von Biomarkern) etc., während mehrerer Phasen bei physiologisch unterschiedlichen Bedingungen, insbesondere während der Schlaf- und Wachphasen. Dabei sind die Anzahl und die zeitlichen Grenzen dieser Phasen nicht oder nur ungenau bekannt.

Aus der Kenntnis dieser Mittelwerte und ggf. deren Anzahl werden abgeleitet:

■ Grenzen der zugehörigen Zeitabschnitte;
■ Parameter zur Quantifizierung der zirkadianen Variation als Mittel zur Erkennung und Überwachung kardialer Erkrankungen;
■ Parameter zur Quantifizierung des Schlafverhaltens eines Patienten als Mittel zur Erkennung und Überwachung von Schlafstörungen.

Hierfür wird zumindest ein Teil des Datenvolumen, das bei der Messung von physiologischen Variablen $X(t)$ gewonnen wird, ausgewertet, wobei Start- und/oder Endpunkte der Zeitabschnitte, d. h. beispielsweise die Lage und Dauer der Schlaf- und Wachphasen, nicht bekannt sind. Vorzugsweise wird das gesamte Datenvolumen für die Auswertung verwendet.

Einfache Beispiele für die so abgeleiteten Parameter sind die Differenz zwischen Tag- und Nacht-Herzrate, die bei herzinsuffizienten Patienten vermindert ist. Anzahl und Länge von Tief- und REM-Schlafphasen lassen Rückschlüsse auf den Schlafrhythmus des Patienten zu und erlauben somit z. B. die Erkennung von Schlafapnoe. Die abgeleiteten Parameter dienen also als Indikatoren für den Gesundheitszustand des Patienten.

Mit Kenntnis der genauen Grenzen der analysierten Zeitabschnitte, z. B. der Grenzen zwischen Schlafen und Wach-Sein, können geeignete Datensegmente für die weitere Datenanalyse mit anderen Methoden identifiziert werden. Beispielsweise ist es damit möglich, die Signalanalyse auf die Schlafphase zu beschränken.

Ferner lassen sich durch die Ergebnisse des vorgestellten Algorithmus das Verhalten des aktiven Implantats besser an den Schlafrhythmus des Implantatträgers anpassen.

Die Erfindung wird nachfolgend unter Bezugnahme auf die Figuren der Zeichnungen an mehreren Ausführungsbeispielen näher erläutert. Es zeigen:

FIG. 1    Beispiel einer Zeitreihe aus RR-Intervallen über 24h;

FIG. 2    die aus der Zeitreihe in Figur 1 hergeleitete Funktion $g(x|\tau)$ der mittleren Zielzustände;

FIG. 3    Darstellung der aus dem $g(x|\tau)$-Plot bestimmten Parameter in der Zeitreihe $X(t)$ aus Figur 1,

FIG. 4    Veranschaulichung von aus dem $g(x|\tau)$-Plot (Figur 2) ermittelten Parametern,

FIG. 5 bis 8    g-Plot von vier verschiedenen Patienten 1 bis 4,

FIG. 9 bis 12    Gegenüberstellungen von RR-Intervallen 910, erfindungsgemäß rekonstruierten Schlaf-/Wachphasen 920 und Phasen gemäß Annotationen 930, und

FIG. 13 bis 21    Gegenüberstellung folgender Daten von acht Probanden:

1. Panel: g-Plot und die Gerade $y = x$ (1310, linke Skala), relative Häufigkeit der RR-Intervalle (1320, rechte Skala) sowie $\xi_1$ und $\xi_2$ (410, 420, als vertikale Linien)
2. Panel: Zeitreihe der RR-Intervalle 910 sowie $\xi_1$ und $\xi_2$ (310, 320, als horizontale Linien)
3. Panel: Zeitreihe der RR-Intervalle nach Glättung bei Fensterbreite $N = 10$ Schläge 1330 bzw. $N = 300$ Schläge 1340
4. Panel: Relative Häufigkeitsverteilungen 1350, 1360 der geglätteten RR-Intervall-Zeitreihen 1330, 1340

In den beispielhaften Ausführungsbeispielen wird die Herzrate $X(t)$ ausgewertet. Anstelle der Herzrate $X(t)$ kann jedoch auch jede andere (physiologische) Variable, wie etwa die Atmung oder (bio)chemische Signale (z. B. Konzentrationsschwankungen von Biomarkern), ausgewertet werden, deren Verlauf durch zwei oder mehrere Phasen geprägt ist.

**Bestimmung der Mittelwerte von Tag- und Nachtphase**

Bestimmt wird dabei zunächst die Häufigkeitsverteilung des Messsignals

$$P(x) = \#\{X(t){=}x\} \, / \, \#\{X(t)\}.$$

Untersucht werden von nun an Paare von Datenpunkten $\{X(t); X(t{+}\tau)\}$ mit einem festen Abstand $\tau$. Dieser beträgt beispielsweise 1000 Herzzyklen.

Erstellt wird die Funktion $g(x|\tau)$ der mittleren Zielzustände in Abhängigkeit des Ausgangszustandes $x = X(t)$:

$$g(x|\tau) = \langle X(t{+}\tau)\rangle_{X(t)=x}$$

Als Beispiel ist in Figur 2 die aus der Zeitreihe aus Figur 1 berechnete Funktion g zur Zeitdifferenz $\tau = 1000$ Schläge dargestellt. Figur 2 zeigt die aus der Zeitreihe in Figur 1 mit Hilfe der oben definierten Formel bestimmte Funktion der mittleren Zielzustände $g(x|\tau)$. Bei kleinen RR-Intervallen x (im Bereich x = 0,4...0,6s) wird im Mittel auf ein RR-Intervall $g \approx 0,65s$ geregelt. Bei hohen x (x = 0,9...1,1s) ist ein mittleres Zielniveau von $g \approx 0,95s$ zu erkennen. Zwischen diesen beiden Bereichen findet sich eine nahezu lineare Übergangsphase mit Steigung 1.

Die Funktion g zeigt an, auf welche Herzrate innerhalb der durch $\tau$ gegebenen Zeitspanne im Mittel geregelt wird, wenn die Herzrate zuvor x betrug. Bei Daten, die der zirkadianen Variation unterworfen sind, zeigen sich nun zwei ausgeprägte Plateaus der Höhe $\xi_1$ und $\xi_2$, die von x = min(x) bis x = $\xi_1$ bzw. von x = $\xi_2$ bis x = max(x) reichen. Verbunden sind diese beiden Plateaus durch einen Übergangsbereich, in dem die Daten typischerweise eine Steigung von 1 aufweisen.

Aus diesem Grund wird die folgende Fitfunktion $y_1(x)$ mit den zwei freien Parametern $\xi_1$ und $\xi_2$ an die Daten angepasst:

$$y_l(x) = \begin{cases} \xi_1 & \text{falls } x < \xi_1\,, \\ x & \text{falls } \xi_1 < x < \xi_2\,, \\ \xi_2 & \text{falls } \xi_2 < x. \end{cases}$$

Dabei wird der $\chi^2$-Wert zusätzlich in geeigneter Weise in Abhängigkeit der Häufigkeitsverteilung P(x) gewichtet. Als vorteilhaft erweist sich z. B. die Gewichtungsfunktion

$$w(x) = \begin{cases} P(x) & \text{falls } P(x) < w_{max} \\ \\ w_{max} & \text{sonst}\,, \end{cases}$$

wobei $W_{max}$ den Maximalwert von w(x) darstellt, der z. B. als Anteil des maximalen P(x) oder in Abhängigkeit der Anzahl der x-Bins definiert wird. Die Funktion w(x) gewichtet die seltenen x-Werte geringer als die übrigen, um so Artefakte durch g-Werte auf geringer Datenbasis zu vermeiden, vermeidet aber gleichzeitig eine zu große Dominanz einzelner g-Werte, indem für alle P(x) oberhalb der Schwelle eine Gleichverteilung verwendet wird.

Weitere mögliche Modifikationen sind die Einschränkung des x-Bereichs, auf dem $\chi^2$ berechnet wird, oder die Verkleinerung des Intervalls, auf dem die Fitfunktion y(x) = x ist, von [$\xi_1$; $\xi_2$] auf [$\xi_1$+dx; $\xi_2$-dx].

Die optimalen Fitparameter $\xi_1$ und $\xi_2$ stellen dann die Mittelwerte der Tag- bzw. Nachtphase dar.

**Bestimmung der Grenzen von Tag- und Nachtphase**

Aus den Mittelwerten der Tag- bzw. Nachtphase lassen sich in den folgenden Schritten die Grenzen der Phasen bestimmen.

Definition eines Schwellwertes z, z. B.

$$z = \xi_2 - \tfrac{1}{4}\cdot(\xi_2 - \xi_1).$$

Abbildung des Herzratensignals X(t) auf eine Symbolfolge S(t) mittels

$$S(t) = \begin{cases} 0 & \text{falls } X(t) < z \qquad (\text{„Tagschlag“}) \\[2em] 1 & \text{sonst} \qquad\qquad (\text{„Nachtschlag“}) \end{cases}$$

Bestimmung der Grenzen einer zusammenhängenden Nachtphase, z. B. mittels:

- Optimierung der Grenzen $T_1$ und $T_2$, sodass die Anzahl der Nachtschläge in der Nachtphase und die Anzahl der Tagschläge in den zwei Phasen vor und nach dem Nachtintervall maximal sind, d. h. $\chi(T_1, T_2)$, definiert durch

$$\chi(T_1, T_2) = \chi_1(T_1) + \chi_2(T_1, T_2) + \chi_3(T_2)$$

mit:

$$\chi_1(T_1) = \#\{S(t) = 0 \mid t < T_1\} \; / \; \#\{S(t) \mid t < T_1\}$$

$$\chi_2(T_1, T_2) = \#\{S(t) = 1 \mid T_1 < t < T_2\} \; / \; \#\{S(t) \mid T_1 < t < T_2\}$$

$$\chi_3(T_2) = \#\{S(t) = 0 \mid T_2 < t\} \; / \; \#\{S(t) \mid T_2 < t\}$$

wird bzgl. der freien Parameter $T_1$ und $T_2$ maximiert; und

- Verwendung der größten zusammenhängenden Gruppe von "Nachtfenstern", d. h. von Fenstern einer definierten Breite, z. B. 5min, in denen die Anzahl der Nachtschläge größer als die der Tagschläge ist. Dabei können auch andere Verhältnisse der Anzahlen verwendet werden, wodurch sich die Sensitivität des Algorithmus und/oder evtl. die Tiefe des Schlafes, aber der eine Phase als Schlafphase identifiziert wird, steuern ließe. Letztgenannte Möglichkeit erklärt sich daraus, dass angesichts der Streuung der Herzrate die Häufigkeit von Nachtschlägen in einem Fenster in Zusammenhang steht mit dem Abstand der mittleren Herzrate in diesem Fenster von dem Schwellwert z. Diese Methode kann auch dazu verwendet werden, mehrere Nachtphasen in einer Messung zu identifizieren, ohne dabei deren Anzahl vorzugeben.

Die so erhaltenen Grenzen $T_1$ und $T_2$ von Tag- und Nachtphasen können u. a. dazu dienen, eine weitergehende Datenanalyse auf bestimmte Datensegmente zu beschränken, z. B. auf die Schlafphase. Ein solches Vorgehen ist deshalb interessant, weil in der Schlafphase die körperliche Belastung bei allen Patienten vergleichbar ist oder weil der Vagotonus besonders hoch ist.

Analog zu der Definition von z können auch zwei Schwellwerte definiert werden, z. B. $Z_1 = \xi_1 + \frac{1}{4} \cdot (\xi_2 - \xi_1)$ und $z = \xi_2 - \frac{1}{4} \cdot (\xi_2 - \xi_1)$, mit denen ein Tag-, ein Nacht- sowie ein dazwischen liegender Übergangsbereich definiert werden. Entsprechend erweitert sich der Wertebereich der Symbolabbildung S um eins. Neben den Mittelwerten $\xi_2$ und $\xi_1$ können in die Definition der Schwellwerte auch z. B. Streuungsmaße eingehen, z. B. die Varianz der gesamten Zeitreihe oder die in einem Fenster berechnete Varianz in Abhängigkeit des zugehörigen Mittelwerts.

## Ableitung von diagnostisch relevanten Parametern

Folgende Parameter geben Hinweise auf den Herzzustand des Patienten:

- Tag-Mittelwert $\xi_1$
- Nacht-Mittelwert $\xi_2$
- Differenz $(\xi_2 - \xi_1)$ zwischen Tag- und Nacht-Mittelwert
- Anzahl der Schläge in der Nachtphase, d. h. $\#\{X(t) \,|X(t) > \xi_2 - \frac{1}{4} \cdot (\xi_2 - \xi_1)\}$
- Anzahl der Schläge in der Tagphase
- Länge der Nachtphase
- Länge der Tagphase

Dabei werden die ersten drei direkt aus dem $g(x|\tau)$-Plot bestimmt, die übrigen in Kombination mit der ursprünglichen Zeitreihe X(t).

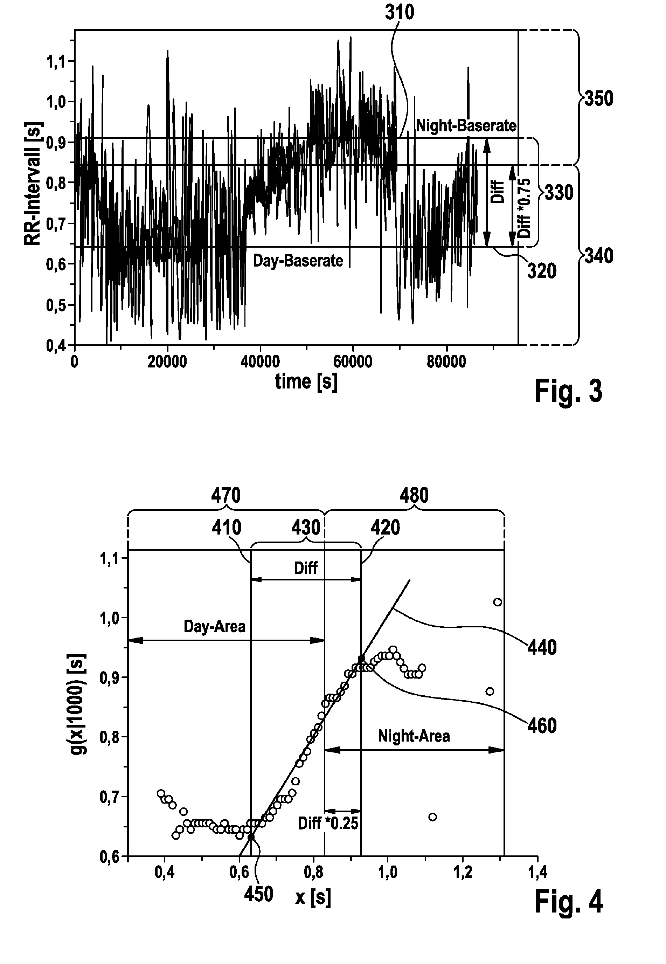

Die aus dem g-Plot bestimmten Parameter sowie ihre Bedeutung innerhalb der Zeitreihe X(t) sind in den Figuren 3 und 4 gezeigt.

In Figur 4 sind die aus dem $g(x|\tau)$-Plot (Figur 2) bestimmten Parameter dargestellt: Die mittleren Herzraten $\xi_1$ und $\xi_2$, in Figur 3 als Day- bzw. Night-Baserate bezeichnet, sind durch vertikale Linien 410, 420 gekennzeichnet (in Figur 3 entsprechen diesen die horizontalen Linien 310, 320). Im Übergangsbereich 430 folgt g der durch eine Linie 440 markierten Geraden mit Steigung 1; bei $x = \xi_1$ und $\xi_2$ ist ein Abknicken der Daten und die Ausbildung der erwähnten Plateauphasen zu erkennen. $\xi_1$ und $\xi_2$ sind durch vertikale Linien 410, 420 gekennzeichnet.

Die Differenz der beiden Baselines $(\xi_2 - \xi_1)$ ist unter der Bezeichnung Diff markiert. Sie ist die Breite des Übergangsbereichs 430.

Mit der Definition des Schwellwertes z werden zwei Bereiche getrennt, die hier durch unterschiedliche Schraffur gekennzeichnet und mit Day- 340, 470 und Night-Area 350, 480 bezeichnet sind. In Figur 3 wird offensichtlich, dass die Night-Area 350 - deutlich an den erhöhten RR-Intervall-Werten zu erkennen - sich dadurch auszeichnet, dass die meisten Schläge sich in der Night-Area 350 befinden.

## Ableitung von diagnostisch relevanten statistischen Maßen

Die Symbolfolge S(t) wurde oben als Mittel zur Bestimmung der Grenzen zwischen Tag-und Nachtphasen eingeführt. Dabei wurde jeder einzelne Schlag anhand einer oder mehrerer Schwellwerte klassifiziert, ob sein Wert typisch für einen Schlaf- oder Wachzustand oder evtl. für eine Übergangsphase sei.

Daneben lassen sich aus S(t) auch statistische Parameter ableiten, die von diagnostischem Nutzen sind. Dazu zählen die Häufigkeitsverteilung von 0 und 1 in der gesamten Datenreihe/in einem Tag- oder Nachtabschnitt/in Fenstern einer definierten Länge. Daraus können Sekundärparameter berechnet werden, z. B. die Anzahl von Fenstern, deren Häufigkeit einer 1 über/unter einer gewissen Schwelle liegt. Des Weiteren sind die Häufigkeitsverteilungen der Längen derjenigen Teilsequenzen von Interesse, die aus einer zusammenhängenden Folge von bloß Nullen bzw. Einsen bestehen.

Derartige Maße geben Informationen über die Tiefe des Schlafes bzw. des Wachzustandes oder über die Häufigkeit einer Schlaf-/Wachphase einer bestimmten Tiefe.

## Erweiterung des Ansatzes zu einer hierarchisch-rekursiven Methode

Die oben beschriebene Methode zielt auf die "große Struktur" der zirkadianen Variation, also auf die Trennung von Merkmalen der Tag- und Nachtphasen ab. Kleinere Strukturen, wie z. B. Tiefschlafphasen, Mittagsschlaf oder Zeiten mit besonders hoher Belastung, werden von den größeren dominiert und dadurch zunächst nicht erfasst.

Diese kleineren Strukturen können ebenfalls erfasst werden, indem die Methode rekursiv angewandt wird. D. h. in einem ersten Durchgang werden Tag- und Nachtphasen getrennt. Im zweiten Durchgang wird eines dieser Datensegmente (oder parallel alle) aufgegriffen und durchlaufen den Algorithmus ein zweites Mal. Das Ergebnis dieses zweiten Durchgangs ist bei einer Tagphase beispielsweise die Identifikation eines Mittagsschlafs oder einer Phase besonders hoher Belastung. Bei einer Nachtphase könnten sich hier unterschiedliche Tiefestufen des Schlafes (leichter-, Tief-, REM-Schlaf) unterscheiden lassen.

Weitere Stufen der Datenverarbeitung sind denkbar; ebenso die Behandlung von Daten, die deutlich kürzer als 24 h sind, wobei dann bei einer geeigneten niedrigeren Stufe begonnen wird.

Bei dieser erweiterten Methode der Datenverarbeitung, in der gestaffelt von größeren zu kleineren Strukturen gegangen wird, spielt die Verzögerung $\tau$ eine bedeutende Rolle, da durch sie die Größe der aufgelösten Zeitintervalle bestimmt wird. Konkret bedeutet dies, dass $\tau$-Werte zwischen 500 und 5000 Schlägen zur Untersuchung der zirkadianen Variation gut geeignet sind; bei kleineren Strukturen wie Schlaftiefe muss $\tau$ verkleinert werden, sodass er unter der typischen Dauer der zu untersuchenden Phänomene liegt.

Unter dieser Einschränkung ist die beschriebene Methode jedoch in einem weiten Bereich von $\tau$ stabil gegenüber Variation von $\tau$, d. h. die Ergebnisse eines entsprechenden Algorithmus zeigen innerhalb dieses Bereiches (im Fall zirkadianer Variation beispielsweise 500 bis 5000 Schläge) wenig Änderung.

Wie schon bei der Ableitung diagnostisch relevanter Parameter aus der Untersuchung der zirkadianen Variation lassen sich weitere Parameter aus der erweiterten Methode bestimmen. Untersucht man z. B. Schlafphasen unterschiedlicher Tiefe, dann lassen sich folgende Fragen beantworten: Welche Schlaftiefen (leichter, mittlerer, tiefer, REM-Schlaf) können identifiziert werden? Wie oft, mit welcher Dauer, in welcher Abfolge treten diese jeweils auf? Aus diesen Informationen ergeben sich damit auch Hinweise für das Vorliegen einer Schlafapnoe.

**Rekonstruktion des Schlafverhaltens in der Nacht**

In einem weiteren Ausführungsbeispiel wird die Erfindung an der Rekonstruktion des Schlafverhaltens in der Nacht näher erläutert.

Ziel der folgenden Datenanalyse ist es, allein aus den RR-Intervall-Zeitreihen eines Patienten Informationen über dessen nächtliches Schlafverhalten zu erhalten. Dabei lassen sich zweierlei Informationen ermitteln: zum einen die mittleren Herzraten in den Schlaf- bzw. Wachphasen, deren Abstand und Absolutwerte einen ersten Indikator für die Tiefe des Schlafs darstellen können. Zum anderen ermöglicht die Kenntnis der beiden mittleren Herzraten die Definition eines Trennwertes, mit dessen Hilfe die Schlaf- und Wachphasen des jeweiligen Patienten rekonstruiert werden können. Um diesen Ansatz zu validieren, wurden bei den im Folgenden vorgestellten Datensätzen die rekonstruierten Phasen mit den Klassifikationen verglichen, die die mit der Datenaufnahme betrauten Ärzte anhand der im Schlaflabor aufgezeichneten Signale vorgenommen hatten. Neben dem vollständigen EKG handelt es sich dabei um Blutdruck, EEG und Atemsignal.

Die Erfindung wurde an den Zeitreihen der MIT-BIH Polysomnographic Database von Physionet getestet. Verwendet wurden die Zeitpunkte der R-Zacke im EKG zur Berechnung der RR-Intervalle sowie die Annotationen, in denen 30s-Intervallen die zugehörigen Schlafphasen zugewiesen sind. Im Folgenden sind die Resultate von vier Beispielpatienten gezeigt.

***Berechnung und Fit des g-Plots***

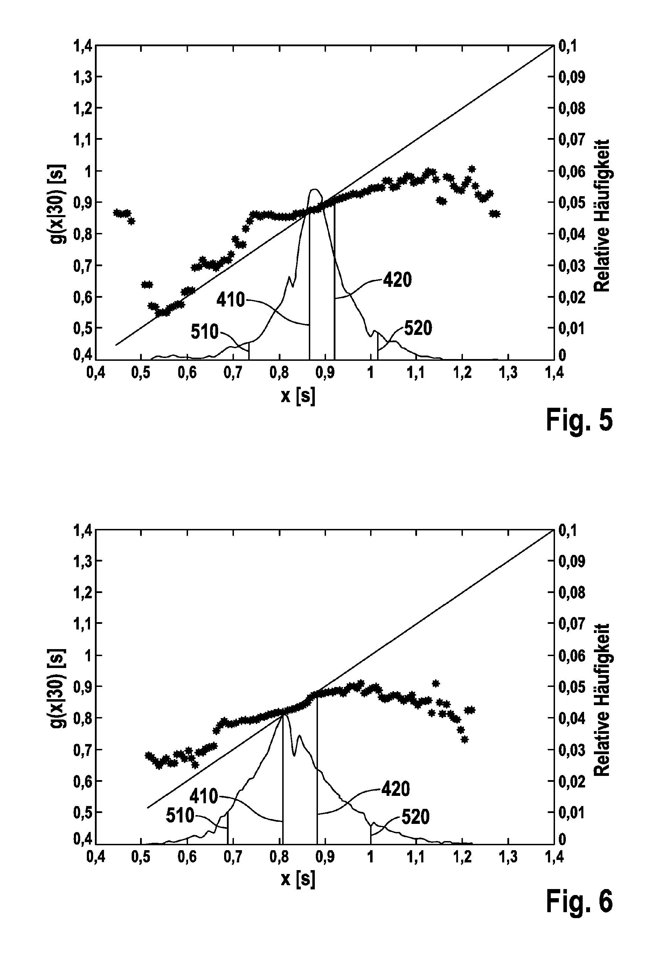

Die Berechnung der g-Plots, die in den Figuren 5 bis 8 gezeigt sind, erfolgte für alle Patienten mit folgenden Parametereinstellungen:

- Verzögerungszeit $\tau$ = 30 Schläge.
- Binbreite = 2/128Hz = 15,625ms. Als Bin wird das Intervall derjenigen Startwerte X(t)=x bezeichnet, über deren zugehörige Nachfolger X(t+$\tau$) bei der Berechnung von g(x|$\tau$) = $\langle X(t+\tau)\rangle_{X(t)=x}$ gemittelt wird und die so einen Punkt im g-Plot ergeben.
- Die Bins sind um eine halbe Binbreite jeweils zueinander versetzt. Der Überlapp der Bins führt dazu, dass jeder Datenpunkt zu zwei Punkten des g-Plots beiträgt.

Der g-Plot ist in Figuren 5 bis 8 durch Sterne mit dem Wertebereich auf der linken Skale dargestellt wurde mit folgender stückweise linearer Funktion gefittet:

$$y_l(x) = \begin{cases} \xi_1 & \text{falls } x < \xi_1 \\ x & \text{falls } \xi_1 < x < \xi_2 \\ \xi_2 & \text{falls } \xi_2 < x \end{cases}$$

Freie Fitparameter waren dabei $\xi_1$ und $\xi_2$, die in den Figuren 5 bis 8 als vertikale Linien 410, 420 dargestellt sind. Die Optimierung erfolgte über das Least-square-Verfahren, wobei jeder g-Datenpunkt mit seiner relativen Häufigkeit gewichtet wurde. Zusätzlich sind in Figuren 5 bis 8 die Gerade y=x (linke Skale) sowie die relative Häufigkeit der RR-Intervalle (rechte Skale) dargestellt.

Beim Fit blieben g-Werte im untersten und obersten 5%-Quantil unberücksichtigt. Die Grenzen der vernachlässigten Bereiche sind in den Figuren durch zwei vertikale Linien 510, 520 gekennzeichnet.

Die Ergebnisse für die vier Patienten lauten:

Patient 1 (Figur 5):

$$\xi_1 = 0{,}87s \qquad \xi_2 = 0{,}92s$$

Patient 2 (Figur 6):

$$\xi_1 = 0{,}80s \qquad \xi_2 = 0{,}88s$$

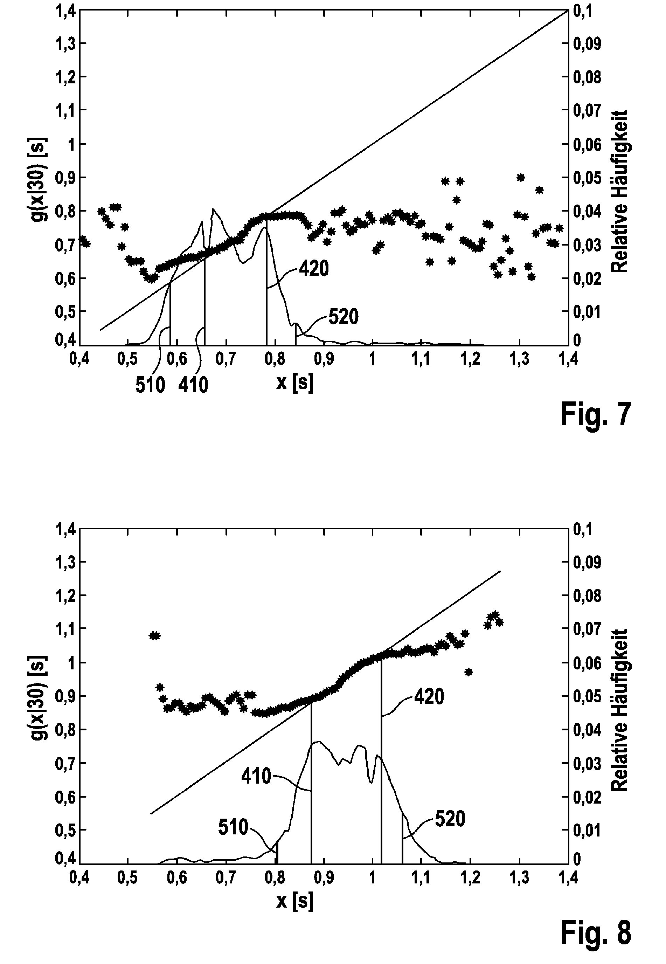

Patient 3 (Figur 7):

$$\xi_1 = 0{,}66s \qquad \xi_2 = 0{,}78s$$

Patient 4 (Figur 8):

$$\xi_1 = 0{,}88s \qquad \xi_2 = 1{,}02s$$

Hinsichtlich alternativer Möglichkeiten, die $\xi$-Werte zu bestimmen, indem zwei Gauß-Funktionen an die Wahrscheinlichkeitsverteilung gefittet werden, wird aus den Figuren 5 bis 8 klar, dass dieser Weg nicht zielführend ist, da die Verteilungen nur in Einzelfällen annähernd bimodal, häufig nur monomodal, bisweilen sogar trimodal oder multimodal sind.

### Rekonstruktion der Schlafphasen

Aus den $\xi$-Werten, die im vorangegangenen Abschnitt berechnet wurden, wurde ein Schwellwert z zur Trennung der Schlaf- und Wachphasen definiert als das arithmetische Mittel der beiden $\xi$-Werte:

Patient 1:     z = 0,88s

Patient 2:     z = 0,84s

Patient 3:     z = 0,72s

Patient 4:     z = 0,95s

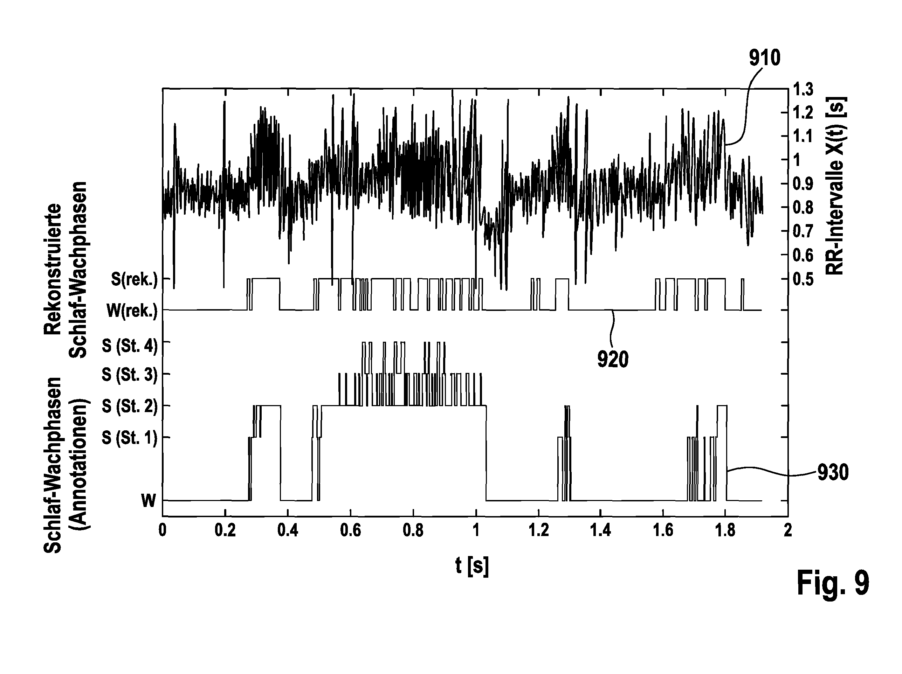

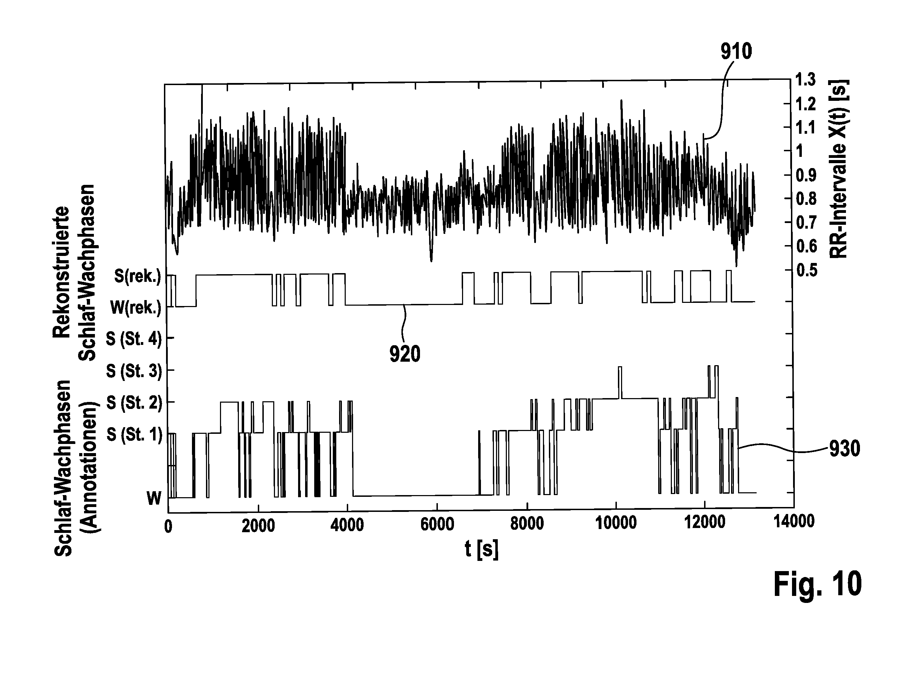

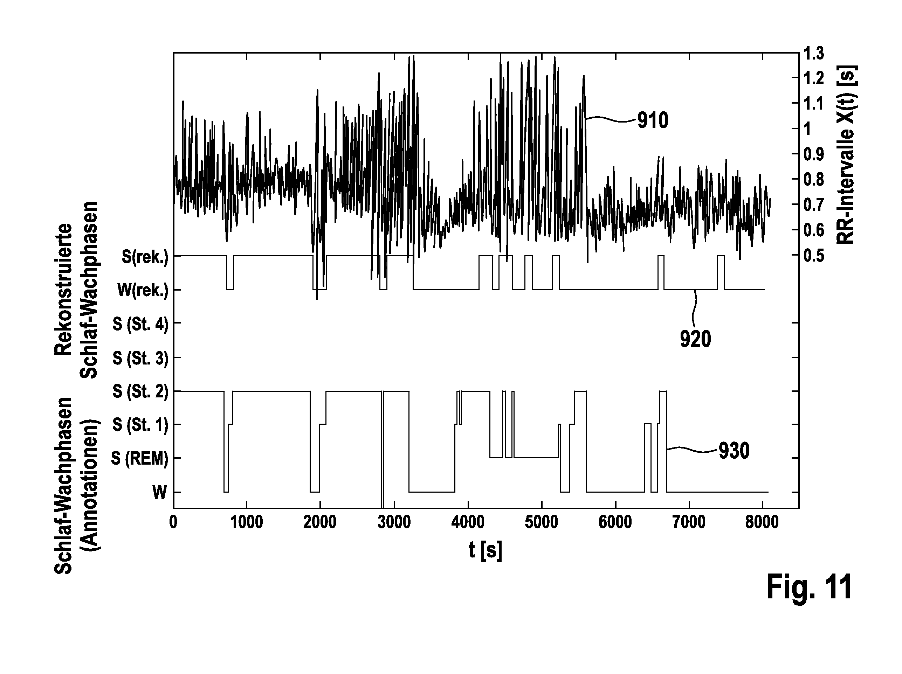

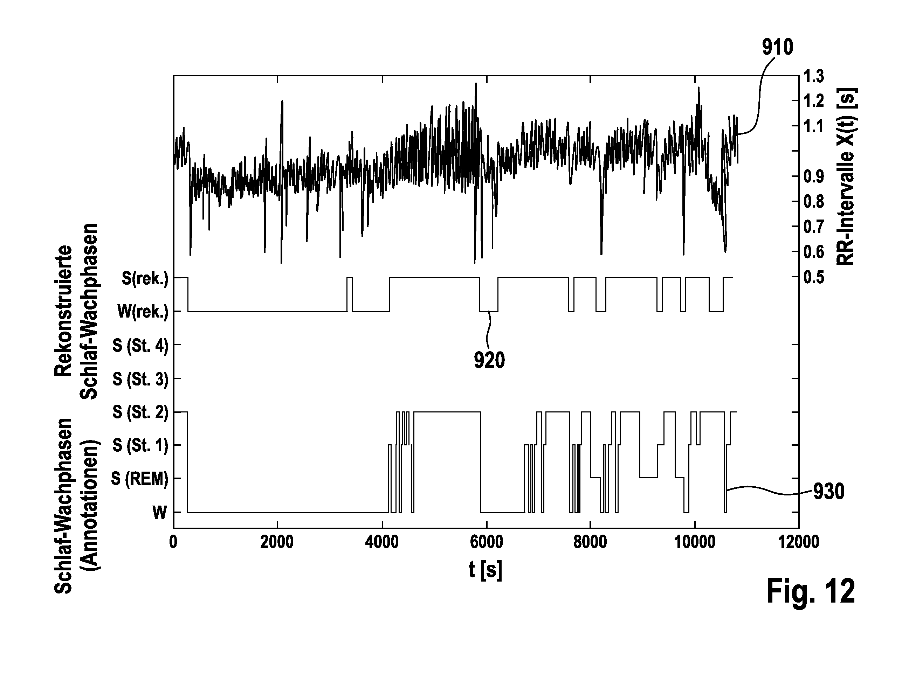

Die Zeitreihe wurde in disjunkte Fenster von 90s Breite unterteilt. In jedem dieser Fenster wurde getestet, ob sich darin mehrheitlich RR-Intervalle größer bzw. kleiner als z befinden, und entsprechend der Wert S (schlafend) bzw. W (wach) zugewiesen (vgl.o.: Beschreibung der Klassifikation). Die ursprünglichen RR-Intervalle 910, die rekonstruierten Schlaf-/Wachphasen 920 sowie die Phasen 930 gemäß Annotationen sind in den Figuren 9 bis 12 dargestellt.

Der g-Plot stellt eine Möglichkeit dar, die Mittelwerte von Phasen physiologisch unterschiedlicher Bedingungen, hier von Schlaf- und Wachphasen, zu bestimmen, wenn weder deren Anzahl, zeitliche Lage noch die zugehörigen Amplituden bekannt sind. Die Kenntnis der Mittelwerte trägt Information über die Schlaftiefe und kann daher von diagnostischem Nutzen sein.

Ferner lassen sich mit ihrer Kenntnis Schwellwerte für eine Trennung von Schlaf- und Wachphasen definieren. Der Ansatz ist besonders dann hilfreich, wenn keine zusätzlichen Sensorgrößen wie Aktivität genutzt werden können, weil sie entweder nicht vorliegen oder in dem betrachteten Zeitabschnitt zu unspezifisch sind. Die so bestimmten Schwellwerte ermöglichen eine Trennung der Schlaf- und Wachphasen und damit eine Quantifizierung des Schlafverhaltens des Patienten.

Gegenüber herkömmlichen alternativen Herangehensweisen weist die Erfindung folgende Vorteile auf:

1. Gegenüber der Verwendung von Bewegungssensoren:

Da die physische Aktivität eines Menschen während des Schlafs deutlich niedriger liegt als am Tag, reichen in der Regel derartige Sensoren zum Zweck der Phasentrennung nicht aus. Außerdem ist für einen möglichst breiten Anwendungsbereich eine möglichst geringe Anzahl an benötigten Signalen wünschenswert. Weiterhin macht der Bewegungssensor eine patientenindividuelle Kalibration notwendig, da die zu bewegende Masse und die Dämpfungseigenschaften des das Implantat umgebenden Mediums vom jeweiligen Körpergewicht abhängen.

2. Gegenüber der Bestimmung aus den Extrema der Zeitreihe:

Die gezeigten Zeitreihen zeigen, dass die Extrema, hier insbesondere die Minima, nicht repräsentativ für Schlaf- bzw. Wachphasen sind und damit zu Fehleinschätzungen führen können. Um dieses Problem zu umgehen, bietet es sich an, die Zeitreihe zunächst durch Mittelung in einem gleitenden Fenster zu glätten. Allerdings treten in den Zeitreihen nicht nur einzelne Schläge als Ausreißer auf, sondern ganze Zeitintervalle, die deutlich unter/über den dominierenden Herzraten liegen. So ist beispielsweise in Figur 9 ein deutlicher Einbruch der RR-Intervalle über eine Dauer von ca. einer viertel Stunde zu erkennen. Gleichzeitig treten aber Phasen unterschiedlicher Schlaftiefe mit deutlich geringerer Breite auf. Die Konstruktion eines geeigneten Algorithmus für die gleitende Mittelung, der breite "Ausreißerintervalle" eliminiert, sie aber berücksichtigt, wenn sie hinreichend oft auftreten, ist also problematisch.

3. Gegenüber der Bestimmung aus der Häufigkeitsverteilung mittels Fit:

Dieser Ansatz basiert auf der Annahme einer bimodalen Verteilung, die nicht immer gegeben ist, wie aus den Figuren 5 bis 8 ersichtlich wird.

Die genannten alternativen Verfahren nach dem Stand der Technik zeigen also deutliches Potential für Fehlklassifikationen. Dagegen stellt das erfindungsgemäße Verfahren einen stabilen und einfachen Algorithmus dar, um Informationen über das Schlafverhalten eines Patienten zu erhalten.

Aus den Zeitreihen in den Figuren 9 bis 12 wird klar, dass sich unterschiedliche Schlafphasen auch in der Varianz der Herzrate zeigen. Wie für die RR-Intervalle 910 lassen sich mit dem erfindungsgemäßen Verfahren auch hierfür Schwellwerte bestimmen, sodass sich durch die Ergänzung des Absolutwertkriteriums durch ein Varianzkriterium eine höhere Trennschärfe erzielen lässt. Aber auch dann wird eine Methode benötigt, um einen geeigneten Grenzwert zu definieren, was durch die Erfindung geleistet wird.

**Bestimmung von mittlerer Tag- bzw. Nachtherzrate aus 24h-Zeitreihen von RR-Intervallen**

Die Erfindung betrifft ein Verfahren, aus RR-Intervall-Zeitreihen die mittleren Herzraten zweier physiologisch unterschiedlicher Zustände zu bestimmen. Beispiele für derartige Zustände sind Tagesaktivität - nächtlicher Schlaf, Ruhe - körperliche Belastung oder Schlaf-/Wachzustände in der Nacht. Eine Ausweitung der Methode auf mehr als zwei Zustände ist möglich.

Die Kenntnis der rekonstruierten Herzraten kann in zweierlei Hinsicht verwendet werden: Erstens stellen sie potentielle diagnostische Marker dar oder/und erlauben die Ableitung sekundärer diagnostisch relevanter Parameter.

So lassen sich beispielsweise über die mittlere Herzrate während der Nacht gesunde Probanden von CHF-Patienten mit NYHA-Klasse I trennen. Zweitens kann die Kenntnis, welchen mittleren Wert die Herzrate in den zwei Zuständen jeweils besitzt, dazu verwendet werden, die Zustände zeitlich zu trennen. So lassen sich bei 24h-Daten Tag und Nacht unterscheiden, bei Nachtaufzeichnungen Schlaf- von Wachphasen trennen oder es lassen sich Zeiten körperlicher Belastung identifizieren. Auch diese Information kann mehrfach verwendet werden: Entweder direkt diagnostisch (z. B. als Maß für das Schlafverhalten des Patienten) oder als Mittel zur Datenvorverarbeitung, sodass andere Verfahren gezielt auf Bereiche in ausschließlich einem physiologischen Zustand angewandt werden können.

Das erfindungsgemäße Verfahren weist mehrere Vorteile auf: Es benötigt keine zusätzlichen Sensordaten, insbesondere nicht von Bewegungs- oder ähnlichen Aktivitätssensoren. Es macht keine Vorannahmen über die Anzahl oder zeitliche Lage/Länge der Intervalle, in denen sich der Patient in einem der beiden Zustände befindet. Beispielsweise ist die Bestimmung von Tag- und Nacht-Herzrate unempfindlich gegenüber nächtlichen Wachphasen, Mittagsschlaf, variablen Zeitpunkten des Einschlafens und Aufstehens, Zeitverschiebung durch Sommer-/Winterzeit oder Reisen, Änderungen im Lebenswandel etc.

Mit dem folgenden Beispielen soll näher erläutert werden, dass die Bestimmung der mittleren Herzraten von Tag und Nacht aus 24h-Daten stabil ist gegenüber einer nicht-bimodalen Häufigkeitsverteilung, gegenüber Artefakten und gegenüber Ausreißerintervallen - also Phänomenen, die für alternative Verfahren Risiken bergen.

Zu diesem Zweck wurden die Datensätze der Normal Sinus Rhythm RR Interval Database von PhysioNet analysiert. Im Folgenden werden repräsentative Beispiele gezeigt.

**g-Plot: Berechnung des Plots und Fit**

Die Berechnung der g-Plots erfolgte für alle Datensätze mit folgenden Parametereinstellungen:

- Verzögerungszeit $\tau$ = 5000 Schläge.
- Binbreite = 2/128Hz = 15,625ms. Als Bin wird das Intervall derjenigen Startwerte X(t)=x bezeichnet, über deren zugehörige Nachfolger X(t+$\tau$) bei der Berechnung von g(x|$\tau$) = $\langle (X(t+\tau) \rangle_{X(t)=x}$ gemittelt wird und die so einen Punkt im g-Plot ergeben.
- Die Bins sind um eine halbe Binbreite jeweils zueinander versetzt.

An den g-Plot wurde folgende stückweise lineare Funktion gefittet:

$$y_l(x) = \begin{cases} \xi_1 & \text{falls } x < \xi_1 \\ x & \text{falls } \xi_1 < x < \xi_2 \\ \xi_2 & \text{falls } \xi_2 < x \end{cases}$$

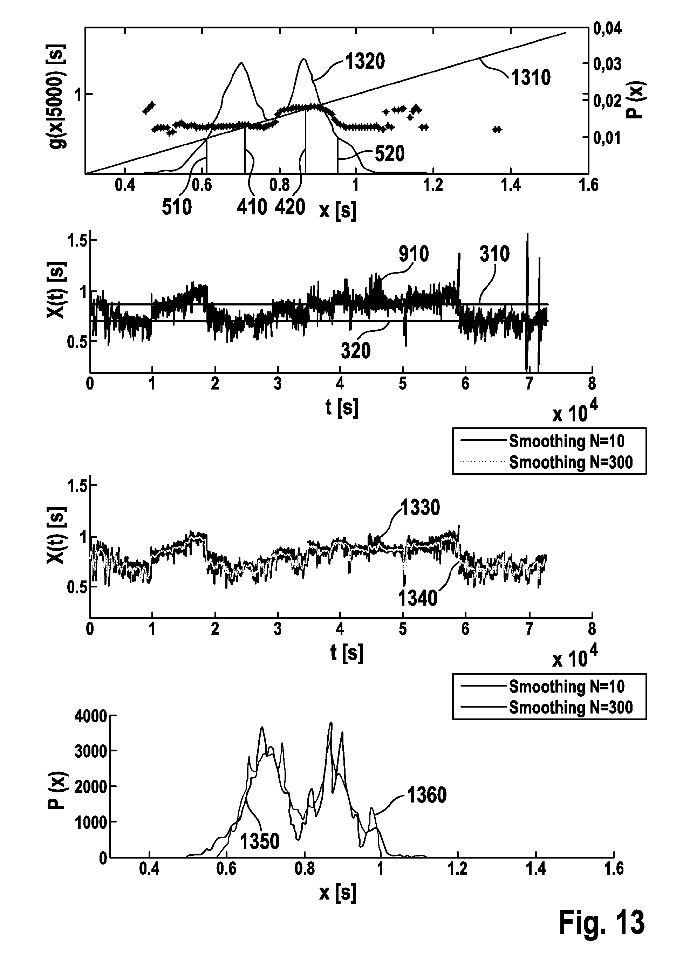

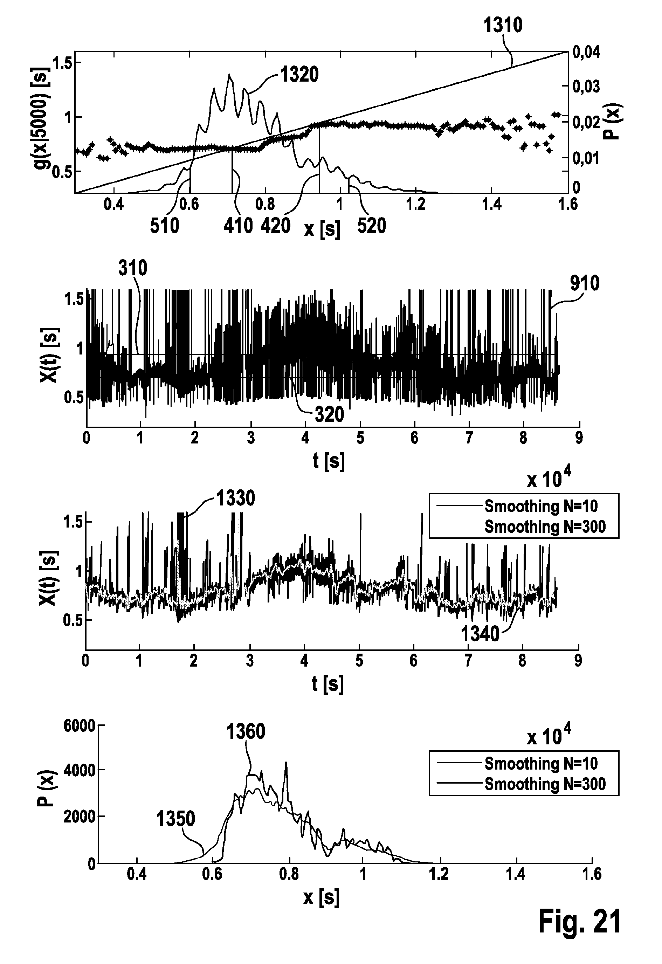

Freie Fitparameter waren dabei $\xi_1$ und $\xi_2$, die in den g-Plots der Figuren 13 bis 21 als vertikale Linien 410, 420 dargestellt sind. Die Optimierung erfolgte über das Least-square-Verfahren, wobei jeder g-Datenpunkt mit seiner relativen Häufigkeit gewichtet wurde.

Beim Fit blieben g-Werte im untersten und obersten 5%-Quantil unberücksichtigt. Die Grenzen der vernachlässigten Bereiche sind in den Abbildungen durch zwei vertikale Linien 510, 520 gekennzeichnet.

Nachfolgend sollen zwei herkömmliche Verfahren mit der Erfindung verglichen werden: Bestimmung der phasenspezifischen Parameter aus den Peaks der Häufigkeitsverteilung der RR-Intervalle 910 oder Bestimmung der phasenspezifischen Parameter aus Extrema der Zeitreihe nach einer Glättung. Um diese beiden Methoden mit dem erfindungsgemäßen Verfahren zu vergleichen, werden in den Figuren 13 bis 21 folgende Daten gezeigt:

1. Panel: g-Plot (Werte dargestellt als Sterne, linke Skala) und die Gerade y = x (1310, linke Skala), relative Häufigkeit der RR-Intervalle (1320, rechte Skala) sowie $\xi_1$ und $\xi_2$ (410, 420, als vertikale Linien)
2. Panel: Zeitreihe der RR-Intervalle 910 sowie $\xi_1$ und $\xi_2$ (310, 320, als horizontale Linien)
3. Panel: Zeitreihe der RR-Intervalle nach Glättung bei Fensterbreite N = 10 Schläge 1330 bzw. N = 300 Schläge 1340
4. Panel: Relative Häufigkeitsverteilungen 1350, 1360 der geglätteten RR-Intervall-Zeitreihen 1330, 1340

**Bimodale Häufigkeitsverteilung**

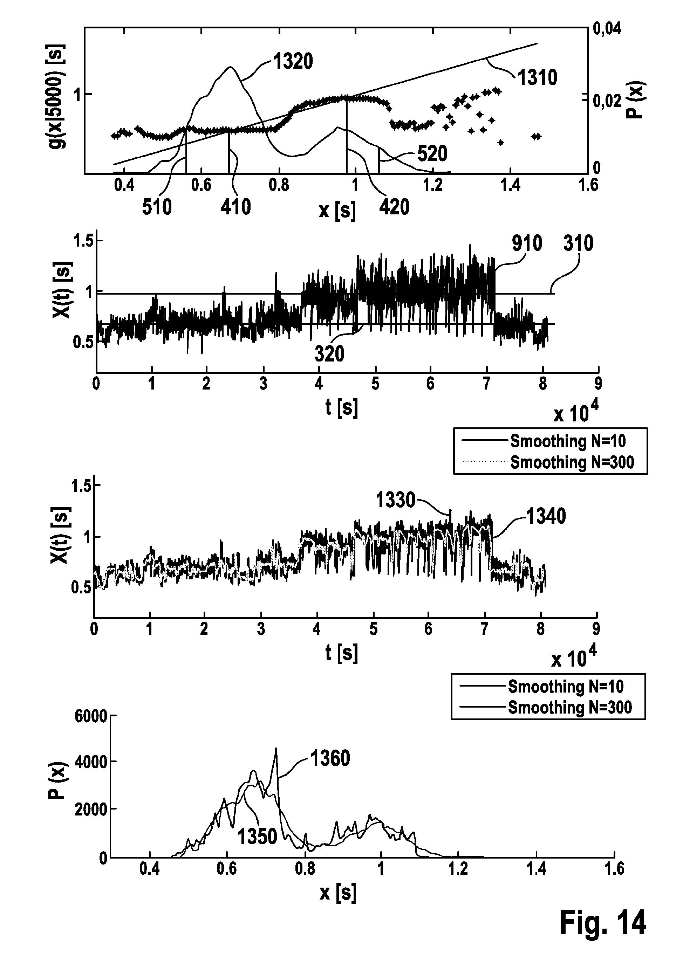

Die beiden in den Figuren 13 und 14 dargestellten Beispiele zeigen die typischerweise für 24h-Daten angenommene bimodale Häufigkeitsverteilung, deren zwei Moden zu den Tag- bzw. Nachtabschnitten der Zeitreihe gehören.

Das erfindungsgemäße Verfahren erkennt die zwei zu Tag und Nacht gehörenden Moden. Die drei verglichenen Methoden liefern ähnliche Ergebnisse. Allerdings unterschätzt die Extremwert-Methode in Figur 14 mit 0,5s den Tag-Mittelwert etwas. Die Histogramme 1350, 1360 der geglätteten Zeitreihen werden mit zunehmender Fenstergröße sehr zerklüftet.

**Monomodale Häufigkeitsverteilung**

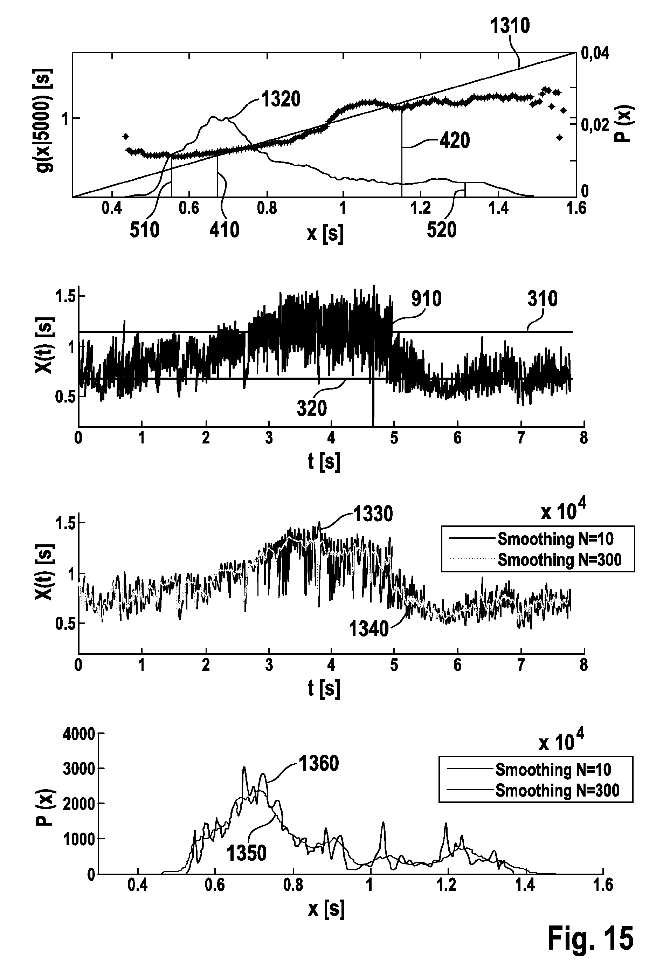

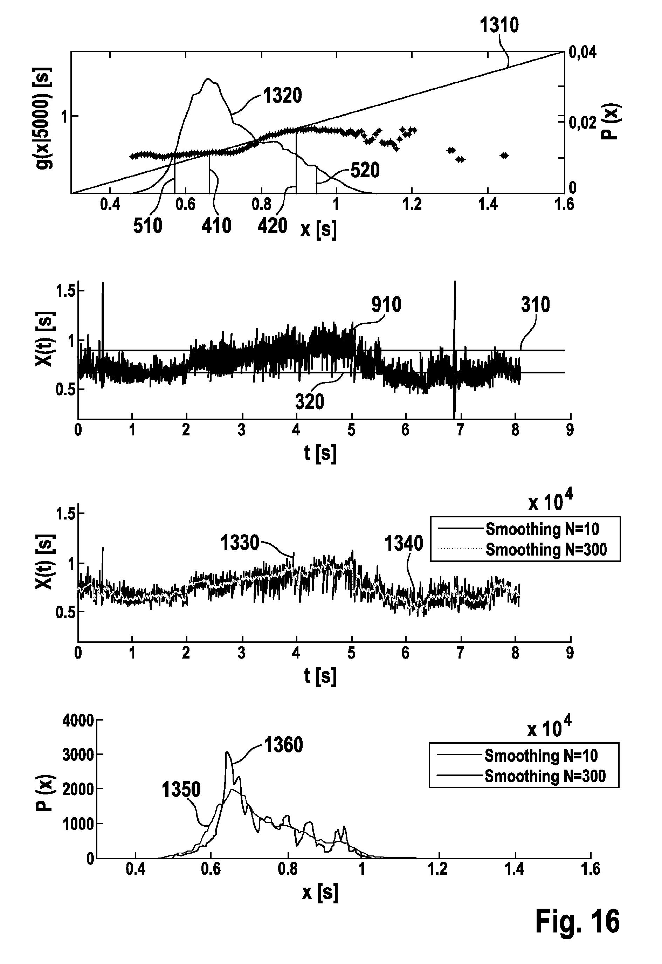

Bei den in den Figuren 15 und 16 dargestellten Beispielen ist trotz ausgeprägter zirkadianer Variation die Bimodalität nur sehr schwach ausgeprägt.

Die hier vorgestellten Zeitreihen weisen nahezu monomodale Häufigkeitsverteilungen auf. Eine Bestimmung der Nachtrate aus den Histogrammen 1320, 1350, 1360 - durch Detektion lokaler Maxima oder Fitten zweier Gauß-Funktionen - ist in diesen Fällen sehr instabil und damit fehleranfällig.

**Tri- und multimodale Häufigkeitsverteilung**

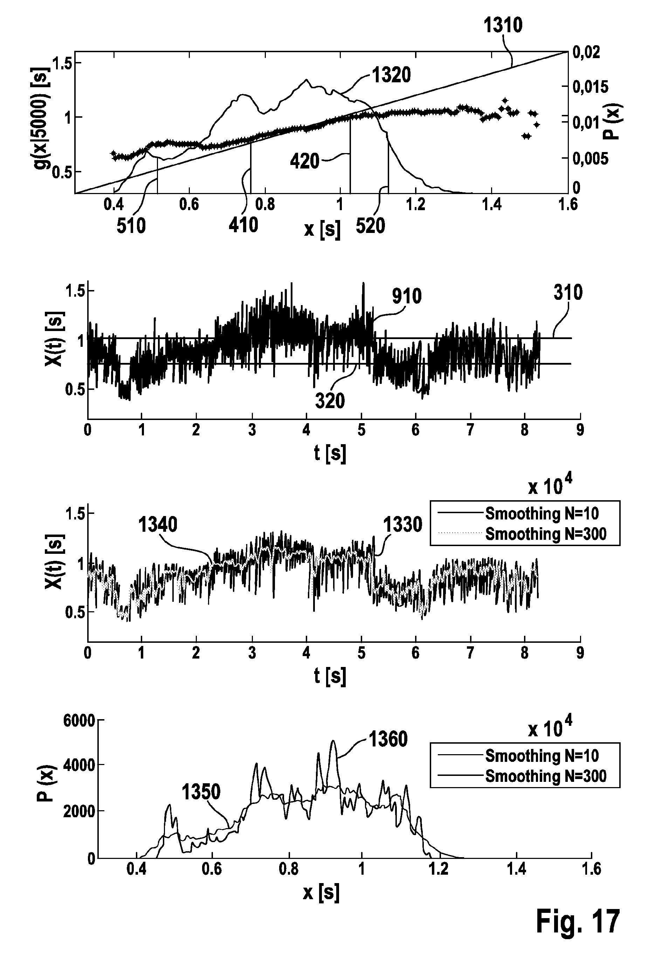

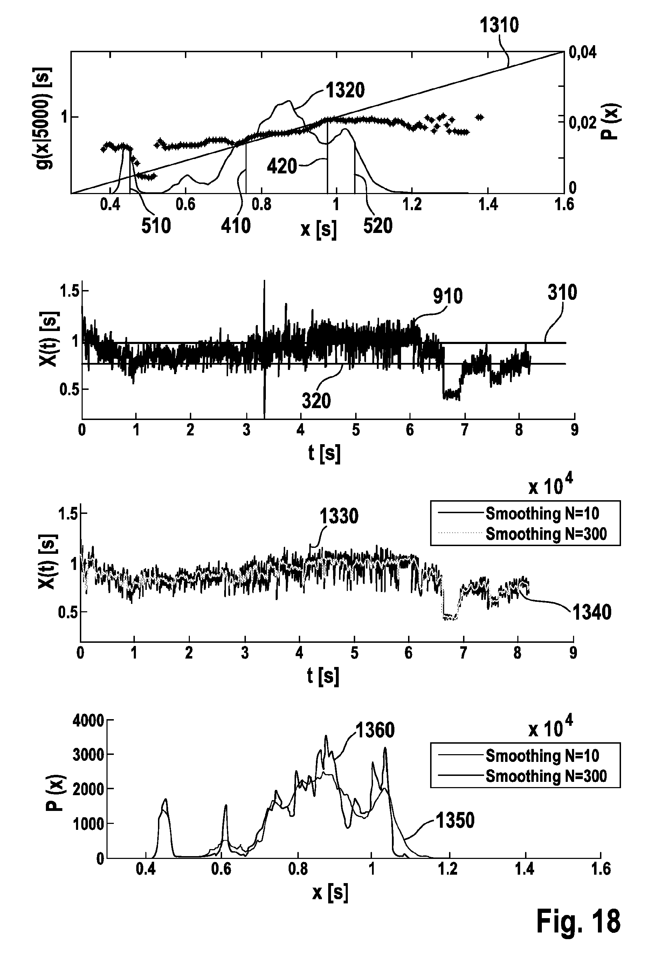

Die in den Figuren 17 und 18 dargestellten Beispiele weisen eine deutliche zirkadiane Variation auf, besitzen aber auch Phasen besonders hoher körperlicher Aktivität am Tage, was sich in der Häufigkeitsverteilung in einer Tri-, in Figur 18 sogar einer Multimodalität niederschlägt.

Die hier vorgestellten Beispiele zeigen deutlich die Grenzen der Alternativverfahren über Extremwertbestimmung bzw. Fitten zweier Gauß-Funktionen im Histogramm 1320, 1350, 1360 auf. Für letzteres ist besonders in Abb. 18 zu erkennen, dass das am stärksten ausgeprägte Maximum weder der Tag- noch der Nachtrate korrespondiert; die lokalen Maxima im Histogramm 1320, 1350, 1360 entsprechen also nicht zwingend den gesuchten Werten für $\xi_1$ und $\xi_2$. Selbst wenn dies der Fall sein sollte, wird die Auswahl der korrekten Maxima aber problematisch: Das Fitten von zwei Gauß-Funktionen wird instabil; die Optimierung wird entweder den Großteil der Verteilung durch einen einzelnen Gauß abdecken und den verbleibenden praktisch zufällig setzen oder/und die beiden Gauß-Funktionen mit möglichst großem Abstand platzieren, um eine möglichst große Abdeckung des Histogramms zu erreichen.

Wird auf einen Fit verzichtet, dann werden für die Auswahl der zwei genutzten lokalen Maxima zusätzliche Kriterien nötig. In jedem Fall benötigen die Verfahren, die $\xi_1$ und $\xi_2$ aus einem der Histogramme 1320, 1350, 1360 bestimmen, Korrekturschritte, die die Methoden verkomplizieren.

Auch die Glättung der Daten - wie vorgeschlagen - bringt keine Abhilfe. Eine Verbesserung der Histogramme 1350, 1360 hin zu einer stärker ausgeprägten Bimodalität ist nicht zu beobachten. Ferner ist auch die Extremwert-Methode nicht zielführend, da Phasen hoher körperlicher Aktivität über ca. eine Stunde, wie sie bei Proband 6 (Figur 18) zu erkennen sind, auch mit sehr langen gleitenden Fenstern kaum wegzumitteln sind. Sie führen aber zu einer deutlichen Unterschätzung des mittleren Tag-RR-Intervalls und verfälschen damit z. B. Kenngrößen, die zu diagnostischen Zwecken den Abstand zwischen Tag- und Nacht-Herzrate verwenden.

**Artefakte**

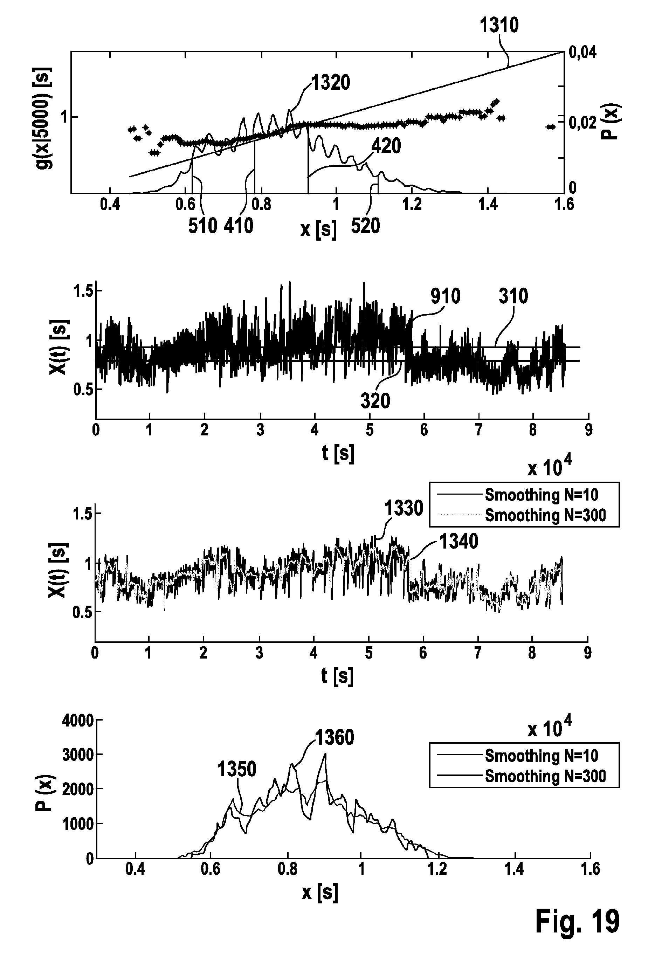

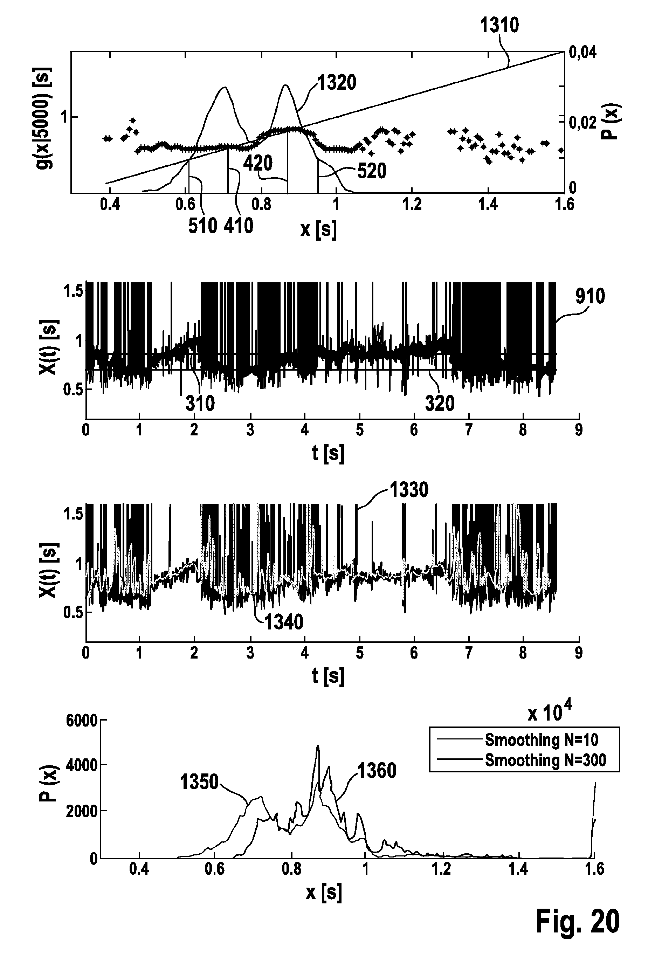

Die in den Figuren 19 bis 21 dargestellten Beispiele demonstrieren, dass das erfindungsgemäße Verfahren auch dann passende Ergebnisse erzielt, wenn die Häufigkeitsverteilung deutliche Ripple aufweist oder wenn die Zeitreihe viele Artefakte oder Extrasystolen enthält. (In Figur 20 sind die Daten dargestellt, die auch Figur 13 zugrundelagen - in Figur 20 jedoch ungefiltert.)

Die Figuren 13 bis 21 zeigen, dass das erfindungsgemäße Verfahren stabil ist gegenüber Abweichungen von den typischerweise von 24h-RR-Intervall-Zeitreihen erwarteten Eigenschaften, d. h. gegenüber nicht-bimodalen (mono-, tri- und multimodalen) Häufigkeitsverteilungen, gegenüber Phasen, die vom zirkadianen Verlauf abweichen (Bereiche hoher körperlicher Aktivität) und gegenüber einer hohen Anzahl an Artefakten oder Extrasystolen. Das erfindungsgemäße Verfahren liefert in allen diesen Fällen Ergebnisse, die bei Überprüfung per Inspektion anhand der Zeitreihe als korrekt eingestuft werden können.

Ein herkömmliches Verfahren sieht vor, die mittleren Herzraten aus der Häufigkeitsverteilung der RR-Intervalle zu bestimmen, beispielsweise durch den Fit zweier Gauß-Funktionen oder durch Identifizierung der beiden lokalen Maxima. Gerade im Fall der gezeigten mono- und polymodalen Verteilungen zeigt dieses aber ein hohes Risiko für instabiles Verhalten und Fehlklassifikationen.

Der Vergleich mit geglätteten Daten zeigte, dass auch dieses herkömmliche Verfahren deutliche Nachteile aufweist. Bei den Wahrscheinlichkeitsverteilungen konnte dadurch keine stärkere Bimodalität erzielt werden; vielmehr waren die Histogramme 1350, 1360 stärker zerklüftet. Ebenso schützte Glättung nicht vor Fehlklassifikationen, wenn Tag- und Nacht-Herzrate mit den Extrema der Zeitreihe identifiziert werden.

Darüber hinaus konnte die Stabilität des g-Plot-Verfahrens gegenüber Artefakten und Extrasystolen gezeigt werden, wie sie gerade bei kardiologischen Zeitreihen häufig auftreten.

Das erfindungsgemäße Verfahren über Berechnung und Fitten des g-Plots ist also im Fall von 24h-Daten eine einfache Methode, die mit wenigen Vorannahmen und Parametern auskommt und die vor allem stabil ist gegenüber atypischem Verhalten der Zeitreihe. Diese Eigenschaften sind wichtig für ein Verfahren, das zur Verarbeitung unbekannter Daten eingesetzt werden soll. Insbesondere für eine Verarbeitung in einem Implantat ist ein Eingriff in das Verfahren oder eine Kalibration unrealistisch. Stabilität stellt also eine nicht zu unterschätzende Voraussetzung dar.

Die Erfindung beschränkt sich in ihrer Ausführungsform nicht auf die vorstehend angegebenen bevorzugten Ausführungsbeispiele. Vielmehr ist eine Anzahl von Varianten denkbar, die von der erfindungsgemäßen Anordnung und dem erfindungsgemäßen Verfahren auch bei grundsätzlich anders gearteten Ausführungen Gebrauch macht.

**Patentansprüche**

1. Verfahren zur Ermittlung von phasenspezifischen Parametern einer physiologischen Größe X(t), **dadurch gekennzeichnet, dass**
zumindest für einen Teil der in einem vorgebbaren Zeitraum liegenden Werte x jeweils ein Mittelwert $g(x|\tau)$ aus solchen Werten $X(t + \tau)$ gebildet wird, für deren Vorgänger $X(t) = x$ gilt, wobei $\tau$ einen zeitlichen Abstand beschreibt, und die phasenspezifischen Parametern durch Auswertung des Mittels $g(x|\tau)$ ermittelt werden.

2. Verfahren nach Anspruch 1, **dadurch gekennzeichnet, dass** die phasenspezifischen Parameter durch Auswertung des Mittels $g(x|\tau)$ allein oder durch Auswertung des Mittels $g(x|\tau)$ in Kombination mit der Größe X(t) und/oder weiterer Messgrößen ermittelt werden.

3. Verfahren nach Anspruch 1 oder 2, **dadurch gekennzeichnet, dass** die Ermittlung der phasenspezifischen Parameter die Ermittlung

- mindestens eines phasenspezifischen Mittelwerts $\xi$ der Größe X(t),
- von Grenzen zwischen verschiedenen Phasen der Größe X(t) und/oder
- der Länge von Phasen der Größe X(t)

umfasst.

4. Verfahren nach Anspruch 3, **dadurch gekennzeichnet, dass** die Grenzen einer Phase der Größe X(t) anhand des mindestens einen phasenspezifischen Mittelwerts $\xi$ bestimmt werden.

5. Verfahren nach einem der vorhergehenden Ansprüche, **dadurch gekennzeichnet, dass** eine Klassifikation der Größe X(t) erfolgt durch eine Zuordnung der Werte von X(t) zu einer ermittelten Phase.

6. Verfahren nach Anspruch 5, **dadurch gekennzeichnet, dass** die Grenzen zwischen verschiedenen Phasen der Größe X(t) und/oder die Länge von Phasen der Größe X(t) durch Auswertung von zumindest einem Teil der klassifizierten Werte ermittelt werden.

7. Verfahren nach einem der vorhergehenden Ansprüche, **dadurch gekennzeichnet, dass** die phasenspezifischen Parameter durch rekursive Bildung und Auswertung der Funktion $g(x|\tau)$ ermittelt werden.

8. Verfahren nach einem der vorhergehenden Ansprüche, **dadurch gekennzeichnet, dass** als Zeitraum die Länge einer Phase der Größe X(t) vorgegeben wird.

9. Verfahren nach einem der vorhergehenden Ansprüche, **dadurch gekennzeichnet, dass** die Funktion der Mittelwerte $g(x|\tau)$ durch eine stückweise lineare Funktion $y_1(x)$, durch eine sigmoidale Funktion $y_s(x)$ oder eine andere geeignete Funktion $y(x)$ approximiert und die phasenspezifischen Parametern durch Auswertung der Funktion $y_1(x)$, $y_s(x)$ bzw. $y(x)$ ermittelt werden.

10. Verfahren nach einem der vorhergehenden Ansprüche, **dadurch gekennzeichnet, dass** es sich bei der Größe X(t) um

- die Herzrate,
- die Atmung oder
- (bio)chemische Signale

handelt.

11. Anordnung mit mindestens einem Chip und/oder Prozessor, wobei die Anordnung derart eingerichtet ist, dass ein Verfahren zur Ermittlung von phasenspezifischen Parametern einer physiologischen Größe X(t) gemäß einem der Ansprüche 1 bis 10 ausführbar ist.

12. Anordnung nach Anspruch 11, **dadurch gekennzeichnet, dass** die Anordnung einen Herzschrittmacher, Defibrillator und/oder ein Gerät zur Patientenüberwachung umfasst.

13. Computerprogramm, das es einer Datenverarbeitungseinrichtung ermöglicht, nachdem es in Speichermittel der Datenverarbeitungseinrichtung geladen worden ist, ein Verfahren zur Ermittlung von phasenspezifischen Parametern einer physiologischen Größe X(t) gemäß einem der Ansprüche 1 bis 10 durchzuführen.

14. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren zur Ermittlung von phasenspezifischen Parametern einer physiologischen Größe X(t) gemäß einem der Ansprüche 1 bis 10 durchzuführen.

15. Verfahren, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

**Fig. 1**

**Fig. 2**

Fig. 3

Fig. 4

Fig. 5

Fig. 6

Fig. 7

Fig. 8

Fig. 9

**Fig. 10**

Fig. 11

Fig. 12

Fig. 13

Fig. 14

Fig. 15

Fig. 16

Fig. 17

Fig. 18

Fig. 19

Fig. 20

Fig. 21

Europäisches Patentamt
European Patent Office
Office européen des brevets

## EUROPÄISCHER RECHERCHENBERICHT

Nummer der Anmeldung

EP 11 17 9130

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 98/46128 A1 (HEARTLINK PTY LTD [AU]; STAMFER HANS GEORGE [AU]) 22. Oktober 1998 (1998-10-22) * Seite 9, Zeile 10 - Zeile 19 * ----- | 1-15 | INV. A61B5/024 G06F19/00 |
| A | DA SILVA LEMOS M ET AL: "Altered cardiovascular responses to chronic angiotensin II infusion in aged rats", REGULATORY PEPTIDES, ELSEVIER SCIENCE BV, NL, Bd. 132, Nr. 1-3, 15. Dezember 2005 (2005-12-15), Seiten 67-73, XP027689035, ISSN: 0167-0115 [gefunden am 2005-12-15] * Seite 69, Abschnitt 2.4, erster Absatz * ----- | 1-15 | |

RECHERCHIERTE SACHGEBIETE (IPC)

A61B
G06F

Der vorliegende Recherchenbericht wurde für alle Patentansprüche erstellt

| Recherchenort | Abschlußdatum der Recherche | Prüfer |
|---|---|---|
| Den Haag | 30. November 2011 | Knüpling, Moritz |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 17 9130

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-11-2011

| Im Recherchenbericht angeführtes Patentdokument | Datum der Veröffentlichung | Mitglied(er) der Patentfamilie | Datum der Veröffentlichung |
|---|---|---|---|
| WO 9846128 A1 | 22-10-1998 | BR 9808446 A | 23-05-2000 |
| | | CA 2284553 A1 | 22-10-1998 |
| | | CN 1251974 A | 03-05-2000 |
| | | EP 1014851 A1 | 05-07-2000 |
| | | IL 132186 A | 20-03-2008 |
| | | JP 2001518823 A | 16-10-2001 |
| | | NO 994689 A | 13-12-1999 |
| | | NZ 337833 A | 29-06-2001 |
| | | PL 336149 A1 | 05-06-2000 |
| | | US 6245021 B1 | 12-06-2001 |
| | | WO 9846128 A1 | 22-10-1998 |

EPO FORM P0461

Für nähere Einzelheiten zu diesem Anhang : siehe Amtsblatt des Europäischen Patentamts, Nr.12/82